# EUROPEAN PATENT SPECIFICATION

(11) **EP 4 246 147 B1**
(45) Date of publication and mention of the grant of the patent: **06.05.2026**
(21) Application number: 23162495.8
(22) Date of filing: 17.03.2023
(51) Int. Cl.: G01N 33/92

(54) **METHOD FOR MEASURING STEROL IN LIPOPROTEIN, AND REAGENT FOR MEASURING STEROL IN LIPOPROTEIN**
VERFAHREN ZUR MESSUNG VON STEROL IN LIPOPROTEIN UND REAGENZ ZUR MESSUNG VON STEROL IN LIPOPROTEIN
PROCÉDÉ DE MESURE DE STÉROL DANS UNE LIPOPROTÉINE ET RÉACTIF DE MESURE DE STÉROL DANS UNE LIPOPROTÉINE

(30) Priority: 18.03.2022 JP 2022043939
(43) Date of publication of application: 20.09.2023
(73) Proprietor: SYSMEX CORPORATION, Kobe-shi Hyogo 651-0073 (JP)
(72) Inventor: KUBO, Takuya, Kobe-shi, Hyogo, 651-0073 (JP); NISHIKAWA, Yoichi, Kobe-shi, Hyogo, 651-0073 (JP); UNO, Shinnosuke, Kobe-shi, Hyogo, 651-0073 (JP); HARADA, Amane, Kobe-shi, Hyogo, 651-0073 (JP); MURAKAMI, Katsuhiro, Kobe-shi, Hyogo, 651-0073 (JP); KIM, Jeeeun, Kobe-shi, Hyogo, 651-0073 (JP); KIRIYAMA, Maria, Kobe-shi, Hyogo, 651-0073 (JP); MIWA, Keiko, Kobe-shi, Hyogo, 651-0073 (JP)
(74) Representative: De Clercq & Partners

(56) References cited:
- WO-A1-2012/064501
- JP-A- 2005 300 467
- US-A1- 2017 315 112
- LOMENICK BRETT ET AL: "Identification and characterization of [beta]-sitosterol target proteins", BIOORGANIC & MEDICINAL CHEMISTRY LETTERS, vol. 25, no. 21, 1 November 2015 (2015-11-01), Amsterdam NL, pages 4976 - 4979, XP093065197, ISSN: 0960-894X, DOI: 10.1016/j.bmcl.2015.03.007
- SHUKLA RAVI S. ET AL: "Development of Streptavidin-Based Nanocomplex for siRNA Delivery", MOLECULAR PHARMACEUTICS, vol. 10, no. 12, 2 December 2013 (2013-12-02), US, pages 4534 - 4545, XP093065203, ISSN: 1543-8384, DOI: 10.1021/mp400355q
- SINGH M ET AL: "Lipoplexes with biotinylated transferrin accessories: Novel, targeted, serum-tolerant gene carriers", INTERNATIONAL JOURNAL OF PHARMACEUTICS, ELSEVIER, NL, vol. 321, no. 1-2, 14 September 2006 (2006-09-14), pages 124 - 137, XP027972643, ISSN: 0378-5173, [retrieved on 20060914]
- WATANABE MADOKA ET AL: "Biotinylated lithocholic acids for affinity chromatography of mammalian DNA polymerases [alpha] and [beta]", BIOORGANIC & MEDICINAL CHEMISTRY LETTERS, vol. 12, no. 3, 1 February 2002 (2002-02-01), Amsterdam NL, pages 287 - 290, XP093065207, ISSN: 0960-894X, DOI: 10.1016/S0960-894X(01)00725-9
- NAKAMURA K. ET AL: "Cell Culture and Xenograft-Bearing Animal Studies of Radiolabeled Antisense DNA Carrier Nanoparticles with Streptavidin as a Linker", THE JOURNAL OF NUCLEAR MEDICINE, vol. 48, no. 11, 1 November 2007 (2007-11-01), US, pages 1845 - 1852, XP093065208, ISSN: 0161-5505, DOI: 10.2967/jnumed.106.039339
- BOOTH C ET AL: "Synthesis of novel biotin anchors", TETRAHEDRON, ELSEVIER SIENCE PUBLISHERS, AMSTERDAM, NL, vol. 57, no. 49, 3 December 2001 (2001-12-03), pages 9859 - 9866, XP004322467, ISSN: 0040-4020, DOI: 10.1016/S0040-4020(01)01003-1
- FIET J ET AL: "Plasma 17-OH pregnenolone: comparison of a time-resolved fluoroimmunoassay using a new tracer 17-OH pregnenolone-3-oxyacetyl-biotine with a radioimmunoassay using ^1^2^5I 17-OH pregnenolone-3-hemisuccinate-histamine", STEROIDS, ELSEVIER SCIENCE PUBLISHERS, NEW YORK, NY, US, vol. 66, no. 2, 1 January 2001 (2001-01-01), pages 81 - 86, XP004902367, ISSN: 0039-128X, DOI: 10.1016/S0039-128X(00)00207-5

## Description

### FIELD OF THE INVENTION

The present invention relates to a method for measuring cholesterol in high-density lipoprotein.

### BACKGROUND

In recent years, an index reflecting function of lipoprotein has attracted attention. As a method for examining the function of lipoprotein, for example, methods described in U.S. Patent Application Publication Nos. 2016/0109469 and 2017/0315112 are known. The method measures the ability to uptake cholesterol that is qualitative activity of lipoprotein. These documents have disclose that a lipoprotein's ability to uptake cholesterol can be measured by contacting the lipoprotein and a tagged cholesterol in a sample to form a lipoprotein incorporating the tagged cholesterol esterified by lecithin-cholesterol acyltransferase (LCAT) in the sample and detecting a signal derived from the incorporated tagged cholesterol.

### SUMMARY OF THE INVENTION

In the tagged cholesterol described in U.S. Patent Application Publication Nos. 2016/0109469 and 2017/0315112, a tag is added via a hydrocarbon chain at C17 position of a sterol skeleton, and a hydroxy group at C3 position can be esterified by LCAT. In the methods described in U.S. Patent Application Publication Nos. 2016/0109469 and 2017/0315112, cholesterol in which a tag is added to other sites of the sterol skeleton is not used. As a lipoprotein functional analysis method, there are still few methods for measuring sterol in lipoprotein, and development of a further measurement method is of interest.

The present invention provides a method for measuring sterol in lipoprotein, comprising: forming a complex by contacting with each other: a lipoprotein in a sample; a tagged sterol; and a first capture body that specifically binds to the tag and comprises a labeling substance, the complex comprising: the lipoprotein comprising the tagged sterol; and the first capture body; and detecting a signal generated by the labeling substance comprised in the complex, wherein in the tagged sterol, the tag is added to C3 position of a sterol skeleton,
wherein the tagged sterol is tagged cholesterol and wherein the lipoprotein is high-density lipoprotein, and wherein the tag and the first capture body are a combination selected from an antigen and an antibody that recognizes the antigen, a hapten and an anti-hapten antibody, a peptide or a protein and an aptamer that recognizes them, a ligand and a receptor thereof, an oligonucleotide and an oligonucleotide having a complementary strand thereof, biotin or a biotin analog and a biotin-binding site, a histidine tag and nitrilotriacetic acid that forms a chelate with a nickel ion (Ni-NTA), and glutathione-S-transferase (GST) and glutathione.

In certain embodiments, the tagged cholesterol is represented by the following formula (I): 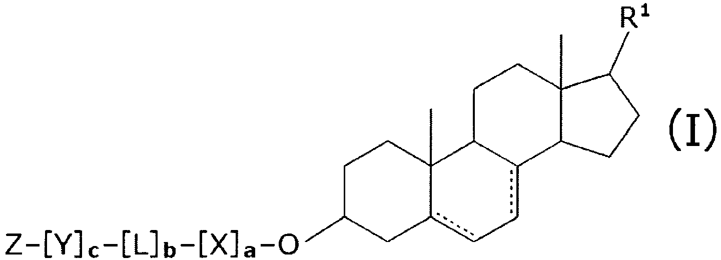 wherein a double line consisting of a solid line and a broken line represents that the bond can be a single bond or a double bond; R¹ is an alkyl group having 1 or more and 6 or less carbon atoms which may have a substituent, or an alkenyl group having 2 or more and 6 or less carbon atoms which may have a substituent; X and Y are identical or different, and are represented by -R²-NH-, -NH-R²-, -R²-(C=O)-NH-, -(C=O)-NH-R²-, -R²-NH-(C=O)-, -NH-(C=O)-R²-, -R²-(C=O)-, -(C=O)-R²-, -R²-(C=O)-O-,-(C=O)-O-R²-, -R²-O-(C=O)-, -O-(C=O)-R²-, -R²-(C=S)-NH-, -(C=S)-NH-R²-, -R²-NH-(C=S)-, -NH-(C=S)-R²-, -R²-O-, -O-R²-, -R²-S-, or -S-R²-, and each R² is independently an atomic bonding, an alkylene group having 1 or more and 10 or less carbon atoms which may have a substituent, an arylene group or heteroarylene group having 6 or more and 12 or less carbon atoms which may have a substituent, or a cycloalkylene group or heterocycloalkylene group having 3 or more and 8 or less carbon atoms which may have a substituent; L is represented by -(CH₂)_{d}-[R³-(CH₂)ₑ]_{f}- or -[(CH₂)ₑ-R³]_{f}-(CH₂)_{d}-, and R³ is an oxygen atom, a sulfur atom, -NH-, -NH-(C=O)-, -(C=O)-NH-, or an atomic bonding; Z is a tag; a and c are identical or different and are an integer of 0 or more and 6 or less; b is 0 or 1; d and e are identical or different and are an integer of 0 or more and 12 or less; and f is an integer of 0 or more and 24 or less.

In certain embodiments, in the formula (I), a is 0 or 1, b and c are 1, X is represented by -NH-(C=O)-R²-, Y is represented by -R²-(C=O)-NH-, R² is each independently an alkylene group having 1 or more and 6 or less carbon atoms and having no substituent, L is represented by -[(CH₂)₂-O]_{f}-(CH₂)_{d}-, d is an integer of 1 or more and 6 or less, and f is an integer of 0 or more and 24 or less.

In certain embodiments, the tag is a biotin group.

In certain embodiments, the tagged cholesterol is represented by following formula (II): 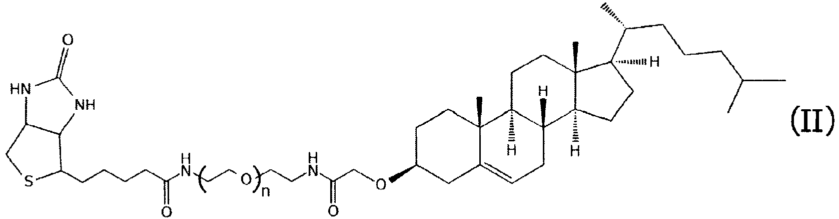 wherein n is an integer of 1 or more and 23 or less,
or following formula (III): 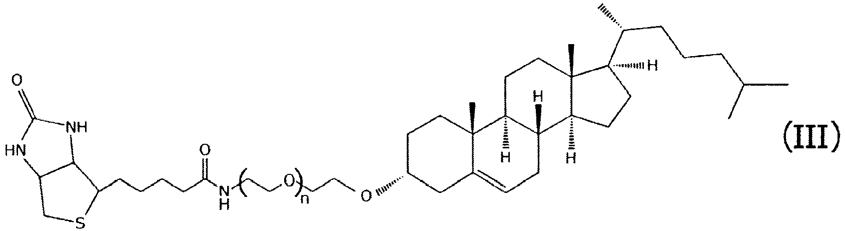 wherein n is an integer of 1 or more and 7 or less,
or following formula (IV): 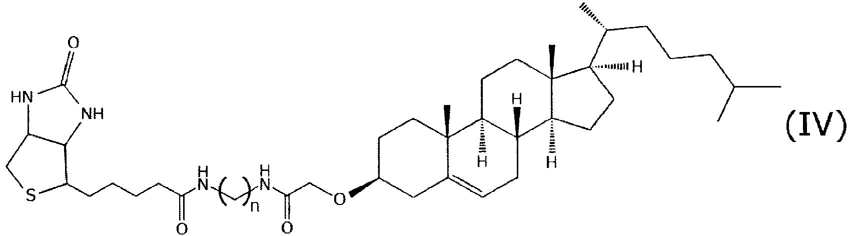 wherein n is an integer of 2 or more and 5 or less.

In certain embodiments, the first capture body is a labeled antibody, labeled avidin, or labeled streptavidin, the first capture body specifically binding to the tag.

In certain embodiments, in the step of forming of the complex, a second capture body that specifically binds to the high-density lipoprotein is further used for contacting the high-density lipoprotein, the tagged cholesterol, and the first capture body with each other.

In certain embodiments, in the step of forming of the complex, the high-density lipoprotein comprising the tagged cholesterol is contacted with the second capture body, and then a complex of the high-density lipoprotein comprising the tagged cholesterol and the second capture body is contacted with the first capture body.

In certain embodiments, in the step of forming of the complex, B/F (bound/free) separation for removing an unreacted free component is performed between one or both of i) the contact between the high-density lipoprotein comprising the tagged cholesterol and the second capture body and ii) the contact between the complex and the first capture body.

In certain embodiments, the second capture body is immobilized on a solid phase, and the complex of the high-density lipoprotein comprising the tagged cholesterol and the second capture body is formed on the solid phase.

In certain embodiments, the second capture body comprises an antibody that specifically binds to the high-density lipoprotein.

In certain embodiments, the B/F separation for removing an unreacted free component is performed between the forming and the detecting step.

### BRIEF DESCRIPTION OF THE DRAWINGS

Fig. 1 is a view showing an example of a reagent for measuring sterol in lipoprotein;
Fig. 2A is a view showing an example of a reagent kit for measuring sterol in lipoprotein;
Fig. 2B is a view showing an example of a reagent kit for measuring sterol in lipoprotein;
Fig. 2C is a view showing an example of a reagent kit for measuring sterol in lipoprotein;
Fig. 2D is a view showing an example of a reagent kit for measuring sterol in lipoprotein;
Fig. 3A is a graph showing results of measuring high-density lipoprotein (HDL) fractions (C0 to C5) with different concentrations using tagged sterol (C5-Amide);
Fig. 3B is a graph showing results of measuring C0 to C5 using tagged sterol (PEG3);
Fig. 3C is a graph showing results of measuring C0 to C5 using tagged sterol (PEG7);
Fig. 3D is a graph showing results of measuring C0 to C5 using tagged sterol (PEG11);
Fig. 3E is a graph showing results of measuring C0 to C5 using tagged sterol (PEG23);
Fig. 4A is a graph showing results of measuring samples containing HDL with different abilities to uptake cholesterol (Sample H and Sample L) using tagged sterol (C5-Amide);
Fig. 4B is a graph showing results of measuring Sample H and Sample L using tagged sterol (PEG3);
Fig. 4C is a graph showing results of measuring Sample H and Sample L using tagged sterol (PEG7);
Fig. 4D is a graph showing results of measuring Sample H and Sample L using tagged sterol (PEG11);
Fig. 4E is a graph showing results of measuring Sample H and Sample L using tagged sterol (PEG23);
Fig. 5A is a graph showing results of measuring Sample H and Sample L using tagged sterol (C2-Amide);
Fig. 5B is a graph showing results of measuring Sample H and Sample L using tagged sterol (PEG3);
Fig. 5C is a graph showing results of measuring Sample H and Sample L using tagged sterol (PEG7);
Fig. 5D is a graph showing results of measuring Sample H and Sample L using tagged sterol (PEG11);
Fig. 5E is a graph showing results of measuring Sample H and Sample L using tagged sterol (PEG23);
Fig. 6A is a graph showing results of measuring C0 to C5 using tagged sterol (PEG1);
Fig. 6B is a graph showing results of measuring C0 to C5 using tagged sterol (PEG2);
Fig. 7A is a graph showing results of measuring Sample H and Sample L using tagged sterol (PEG1);
Fig. 7B is a graph showing results of measuring Sample H and Sample L using tagged sterol (PEG2);
Fig. 8A is a graph showing results of measuring C0 to C5 using tagged sterol (PEG7 (ether));
Fig. 8B is a graph showing results of measuring Sample H and Sample L using tagged sterol (PEG7 (ether));
Fig. 9A is a graph showing results of measuring an rLCAT-added sample (rLCAT+) and an LCAT-unadded sample (rLCAT-) using tagged sterol (C2-Amide);
Fig. 9B is a graph showing results of measuring rLCAT+ and rLCAT- using tagged sterol (PEG3);
Fig. 9C is a graph showing results of measuring rLCAT+ and rLCAT- using tagged sterol (PEG7);
Fig. 9D is a graph showing results of measuring rLCAT+ and rLCAT- using tagged sterol (PEG11);
Fig. 9E is a graph showing results of measuring rLCAT+ and rLCAT- using tagged sterol (PEG23);
Fig. 10 is a graph showing results of measuring an N-ethylmaleimide (NEM)-added sample and an NEM-unadded sample, using tagged sterol (PEG3); and
Fig. 11 is a graph showing results of measuring a sample oxidized with hydrogen peroxide and a sample to which hydrogen peroxide was not added, using tagged sterol (PEG3).

### DETAILED DESCRIPTION OF THE PREFERRED EMBODIMENTS

When naturally occurring cholesterol comes into contact with lipoprotein in vivo, the cholesterol is incorporated into the lipoprotein. Free cholesterol in the blood initially binds to the surface layer (phospholipid membrane) of the lipoprotein. When a hydroxy group at C3 position of cholesterol is esterified by LCAT, lipophilicity is improved, and the cholesterol moves from the surface layer to the center of the lipoprotein particle. On the other hand, a tagged cholesterol used in the method for measuring cholesterol in high-density lipoprotein of the present invention (hereinafter, also referred to as "the measurement method of the present invention") is not esterified by LCAT because the tag is added to C3 position of a cholesterol skeleton. As used herein, the "sterol skeleton" refers to a skeleton represented by the following formula: 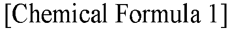 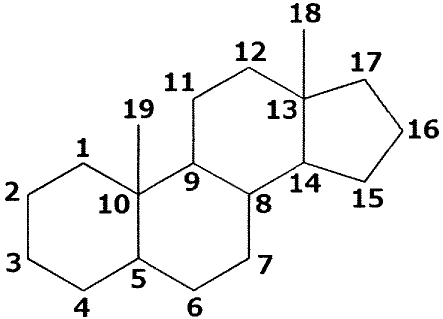 wherein the number represents the position of the carbon in the sterol skeleton.

The surface layer of the high-density lipoprotein in a sample obtained from a living body contains endogenous cholesterol derived from the living body. The sample also includes endogenous LCAT derived from the living body. Endogenous cholesterol in the surface layer of the high-density lipoprotein is esterified by LCAT to produce a cholesterol ester. Then, the cholesterol ester moves into the high-density lipoprotein. The movement of the endogenous cholesterol from the surface layer to the inside gives room for binding of the tagged cholesterol in the surface layer. The tagged cholesterol can contact with LCAT, but as noted above, the tagged cholesterol is not considered to be esterified by LCAT. Therefore, it is considered that the tagged cholesterol remains on the surface layer of the high-density lipoprotein. When the high-density lipoprotein can move more endogenous cholesterol from the surface layer to the inside, it is considered that more tagged cholesterols can bind in the surface layer. When more tagged cholsterols bind in the surface layer of the high-density lipoprotein, a stronger signal is detected from the high-density lipoprotein. Therefore, the signal obtained by the measurement method of the present invention can be an index indicating function of high-density lipoprotein.

When the sample comes into contact with the tagged cholesterol, it is considered that the tagged cholesterol binds to the surface layer of the high-density lipoprotein, and that the tag portion is exposed on the outer surface of the high-density lipoprotein. The "outer surface of the lipoprotein" refers to the outer surface of the lipoprotein particle. The term "exposed on the outer surface" is intended to refer to both existing on the outer surface of the lipoprotein and protruding from the outer surface of the lipoprotein. The tagged cholesterol in the high-density lipoprotein is detected by binding a tag exposed on the outer surface of the high-density lipoprotein containing the tagged cholesterol to a first capture body that specifically binds to the tag. Hereinafter, each step in the measurement method of the present invention will be described.

In the measurement method of the present invention, by contacting with each other the high-density lipoprotein in the sample, the tagged cholesterol, and the first capture body that specifically binds to the tag and has a labeling substance, a complex containing the high-density lipoprotein containing the tagged cholesterol and the first capture body is formed.

The sample is not particularly limited as long as the sample contains high-density lipoprotein. Examples of the sample include blood samples. Examples of the blood samples include blood (whole blood), plasma, serum, and the like. A sample containing lipoprotein may be separated or fractionated by a known method such as ultracentrifugation or polyethylene glycol (PEG) precipitation to obtain a fraction containing high-density lipoprotein. The fraction containing high-density lipoprotein thus obtained may be used as a sample containing high-density lipoprotein.

Lipoproteins may be any of high density lipoprotein (HDL), low density lipoprotein (LDL), medium density lipoprotein (IDL), very low density lipoprotein (VLDL), or chylomicron (CM). HDL is lipoprotein with a density of 1.063 g/mL or more. LDL is lipoprotein with a density of 1.019 g/mL or more and less than 1.063 g/mL. IDL is lipoprotein with a density of 1.006 g/mL or more and less than 1.019 g/mL. VLDL is lipoprotein with a density of 0.95 g/mL or more and less than 1.006 g/mL. CM is lipoprotein with a density of less than 0.95 g/mL.

The sample containing high-density lipoprotein may be diluted. For example, in order to adjust the high-density lipoprotein concentration, the above blood sample or a liquid obtained by diluting a fraction containing high-density lipoprotein with an aqueous medium may be used as a sample. Examples of the aqueous medium include water, physiological saline solutions, buffer solutions, and the like. Examples of the buffer solution include phosphate buffered saline (PBS), Tris-HCl, Good buffers, and the like.

The concentration of apolipoprotein that is a component of lipoprotein is an index of the concentration of lipoprotein in the sample. The high-density lipoprotein concentration in the sample may be adjusted by diluting the sample containing high-density lipoprotein based on the apolipoprotein concentration. The concentration of apolipoprotein can be measured by known immunoassays (for example, immunoturbidimetry). As the apolipoprotein, ApoAI or ApoE is preferred.

A blocking agent may be added to the sample as necessary. Examples of the blocking agent include casein, bovine serum albumin (BSA), cyclic oligosaccharides (for example, cyclodextrin, hydroxypropyl cyclodextrin, and the like), and 2-methacryloyloxyethyl phosphorylcholine polymers (for example, LIPIDURE series manufactured by NOF CORPORATION, particularly LIPIDURE-BL203). A fatty acid required for esterification reaction of cholesterol with lipoprotein or a composition containing the same (for example, liposome) may be added to a sample. Liposomes can be prepared, for example, by mixing dimyristoylphosphatidylglycerol, cholesterol, and hydrogenated soybean phosphatidylcholine.

The tagged cholesterol refers to the cholesterol having a sterol skeleton represented by the above formula, in which a tag is directly or indirectly added to a carbon atom at C3 position thereof. The phrase "a tag is indirectly added to a carbon atom at C3 position thereof" includes addition of a tag to a carbon atom at the C3 position of cholesterol via a linker, addition of a tag via a substituent when the substituent is bonded to the carbon atom at the C3 position of cholesterol, and addition of a tag via a linker to the substituent at the C3 position. Preferably, the tagged cholesterol has a hydrocarbon chain which may have a substituent at C17 position of the sterol skeleton represented by the above formula.

The tagged cholesterol can be prepared by adding a tag to the C3 position of cholesterol. The tag may be added to the carbon atom at the C3 position of cholesterol directly or via a linker. When the substituent is bonded to the carbon atom at the C3 position of cholesterol, the tag may be added to this substituent. Alternatively, the tag may be added to the substituent at the C3 position of cholesterol via a linker. For example, when cholesterol having a hydroxy group at the C3 position is used, the tag may be added to the hydroxy group at the C3 position directly or via a linker. Examples of analogs of cholesterol include epicholesterol, allocholesterol, cholestanol, coprostanol, 7-dehydrocholesterol, stigmasterol, sitosterol, campesterol, α-spinasterol, brassicasterol, 24-methylenesterol, and the like.

Examples of the tagged cholesterol include compounds represented by following formula (I) (hereinafter, also referred to as "tagged cholesterol of formula (I)"). 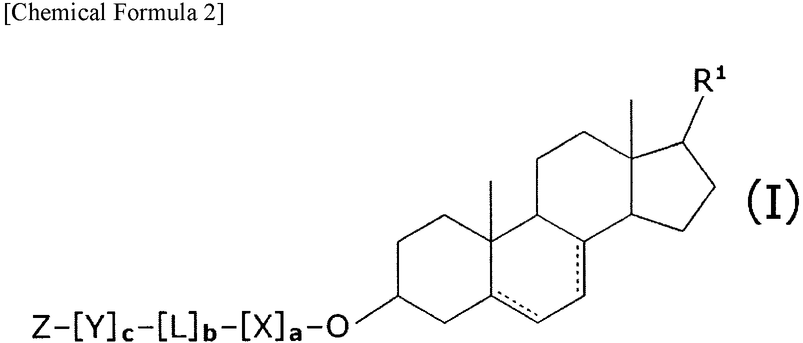 wherein double lines of a solid line and a broken line each independently represent a single bond or a double bond;
R¹ is an alkyl group having 1 or more and 6 or less carbon atoms which may have a substituent, or an alkenyl group having 2 or more and 6 or less carbon atoms which may have a substituent;
X and Y are identical or different, and are represented by -R²-NH-, -NH-R²-,-R²-(C=O)-NH-, -(C=O)-NH-R²-, -R²-NH-(C=O)-, -NH-(C=O)-R²-, -R²-(C=O)-, -(C=O)-R²-, -R²-(C=O)-O-, -(C=O)-O-R²-, -R²-O-(C=O)-, -O-(C=O)-R²-, -R²-(C=S)-NH-,-(C=S)-NH-R²-, -R²-NH-(C=S)-, -NH-(C=S)-R²-, -R²-O-, -O-R²-, -R²-S-, or -S-R²-, and each R² is independently an atomic bonding, an alkylene group having 1 or more and 10 or less carbon atoms which may have a substituent, an arylene group or heteroarylene group having 6 or more and 12 or less carbon atoms which may have a substituent, or a cycloalkylene group or heterocycloalkylene group having 3 or more and 8 or less carbon atoms which may have a substituent;
L is represented by -(CH₂)_{d}-[R³-(CH₂)ₑ]_{f}- or -[(CH₂)ₑ-R³]_{f}-(CH₂)_{d}-, and R³ is an oxygen atom, a sulfur atom, -NH-, -NH-(C=O)-, -(C=O)-NH-, or an atomic bonding;
Z is a tag;
a and c are identical or different and are an integer of 0 or more and 6 or less;
b is 0 or 1;
d and e are identical or different and are an integer of 0 or more and 12 or less; and
f is an integer of 0 or more and to 24 or less.

In the formula (I), a portion represented by "-[X]ₐ-[L]_{b}-[Y]_{c}-" corresponds to a linker connecting the tag and the cholesterol moiety. In the formula (I), when a, b and c are all 0, the tagged cholesterol represented by the formula has no linker. That is, the tag is bound to an oxygen atom at C3 position of the cholesterol moiety (hereinafter, also referred to as "O at C3 position"). In the formula (I), when any one of a, b and c is not 0, the tagged cholesterol of the formula (I) has a linker between the tag and the cholesterol moiety. It is believed that the linker further facilitates the bonding between the tag exposed on the outer surface of the high-density lipoprotein and the first capture body. Each substituent in the formula (I) will be described below.

In the formula (I), the bond between C5 position and C6 position is a double bond, and the bond between C7 position and C8 position is a single bond.

R¹ is a 1,5-dimethylhexyl group. This is the same as alkyl chains at C20 to C27 positions of naturally occurring cholesterol.

When a is an integer of 1 or more, [X]ₐ corresponds to a connecting moiety between O at the C3 position and L, [Y]_{c}, or Z (tag). L corresponds to a spacer and has a linear structure that adds a predetermined length to the linker. When c is an integer of 1 or more, [Y]_{c} corresponds to a connecting moiety between Z (tag) and L, [X]ₐ, or O at the C3 position. X and Y are determined according to the type of reaction that bonds between the cholesterol moiety and the linker and the type of reaction that bonds between the linker and the tag.

In R² and R³, the atomic bonding refers to a direct bonding without intervening any other atom.

When R² is an alkylene group having 1 to 10 carbon atoms, examples of the alkylene group include methylene, ethylene, propylene, isopropylene, butylene, isobutylene, pentylene, neopentylene, hexylene, heptylene, octylene, 2-ethylhexylene, nonylene, and decylene groups. Among them, an alkylene group having 1 or more and 4 or less carbon atoms is preferred. When R² is an alkylene group having a substituent, the above number of carbon atoms does not include the number of carbon atoms in the substituent.

When R² is an arylene group or a heteroarylene group, the group should be an aromatic ring having 6 or more and 12 or less carbon atoms which may contain one or more hetero atoms selected from N, S, O, and P. Examples of the group include phenylene, naphthylene, biphenylylene, furanylene, pyrrolene, thiophenylene, triazolene, oxadiazolene, pyridylene, and pyrimidylene groups. When R² is an arylene group or a heteroarylene group having a substituent, the above number of carbon atoms does not include the number of carbon atoms in the substituent.

When R² is a cycloalkylene group or a heterocycloalkylene group, the group should be a non-aromatic ring having 3 or more and 8 or less carbon atoms which may include one or more hetero atoms selected from N, S, O, and P. Examples of the group include cyclopropylene, cyclobutylene, cyclopentylene, cyclohexylene, cycloheptylene, cyclooctylene, pyrrolidinylene, piperidinylene, and morpholinylene groups. When R² is a cycloalkylene group or a heterocycloalkylene group having a substituent, the above number of carbon atoms does not include the number of carbon atoms in the substituent.

Examples of the substituent in R² include hydroxy, cyano, alkoxy, nitro, =O, =S, -SH, halogen, haloalkyl, heteroalkyl, carboxyalkyl, amine, amide, and thioether groups. R² may have a plurality of the substituents. Halogen represents fluorine, chlorine, bromine, or iodine. Alkoxy represents an -O-alkyl group, and the alkyl group is a linear or branched saturated aliphatic hydrocarbon group having 1 or more and 5 or less carbon atoms, and preferably 1 or 2 carbon atoms.

It is preferable that L has a structure that does not inhibit binding between high-density lipoprotein and cholesterol and has a property that the linker moiety is hardly incorporated into high-density lipoprotein. Examples of such a structure include a structure containing a hydrophilic polymer. For example, in the formula (I), it is preferable that b is 1 and R³ is an oxygen atom. At this time, L is represented by-(CH₂)_{d}-[O-(CH₂)ₑ]_{f}- or -[(CH₂)ₑ-O]_{f}-(CH₂)_{d}-. d is an integer of 0 or more and 12 or less, e is an integer of 1 or more and 6 or less, and f is an integer of 1 to 24. Preferably, d and e are identical or different and are an integer of 1 or more and 4 or less. More preferably, d and e are 2. f is preferably an integer of 1 or more and 23 or less, more preferably an integer of 3 or more and 23 or less, and particularly preferably an integer of 3 or more and 11 or less.

In a preferred embodiment, in the formula (I), a is 0 or 1, b and c are 1, X is represented by NH-(C=O)-R²-, Y is represented by -R²-(C=O)-NH-, R² is each independently an alkylene group having 1 or more and 6 or less carbon atoms and having no substituent, L is represented by -[(CH₂)₂-O]_{f}-(CH₂)_{d}-, d is an integer of 1 or more and 6 or less, and f is an integer of 0 or more and to 24 or less. R¹ is a 1,5-dimethylhexyl group.

For example, in the formula (I), a, b, and c are 1, X is represented by -NH-(C=O)-(CH₂)ₙ- (wherein n is an integer of 1 or more and 6 or less, preferably 2, 3 or 4), Y is represented by -(CH₂)₄-(C=O)-NH-, L is represented by -[(CH₂)₂-O]_{f}-(CH₂)₂-, and f is an integer of 1 or more and 23 or less. Alternatively, in the formula (I), a, b, and c are 1, X is represented by -NH-(C=O)-(CH₂)ₙ- (wherein n is an integer of 1 or more and 6 or less, preferably 2, 3 or 4), Y is represented by -(CH₂)₄-(C=O)-NH-, L is represented by-(CH₂)_{f}-, and f is an integer of 1 or more and 6 or less, preferably an integer of 2 or more and 5 or less. Alternatively, in the formula (I), a is 0, b and c are 1, Y is represented by-(CH₂)₄-(C=O)-NH-, L is represented by -[(CH₂)₂-O]_{f}-(CH₂)₂-, and f is an integer of 1 or more and 23 or less. In any case, R¹ is a 1,5-dimethylhexyl group.

The tag may be any of naturally occurring substances and synthesized substances. Combinations of a tag and a substance capable of specifically binding to the tag are selected from an antigen and an antibody that recognizes the antigen, a hapten and an anti-hapten antibody, a peptide or a protein and an aptamer that recognizes them, a ligand and a receptor thereof, oligonucleotides and oligonucleotide having complementary strands thereof, biotin and its analogs and biotin-binding sites, histidine tag (a peptide containing histidine of 6 to 10 residues) and Ni-NTA (nitrilotriacetic acid that forms a chelate with a nickel ion), glutathione-S-transferase (GST) and glutathione. The antigen as a tag may be a peptide tag and a protein tag known in the art, and examples thereof include FLAG (registered trademark), hemagglutinin (HA), Myc protein, green fluorescent protein (GFP), and the like. Examples of the hapten as a tag include 2,4-dinitrophenyl (DNP) group and the like. As a capture body that specifically binds to DNP, an anti-DNP antibody is suitable.

Examples of the biotin analogs include desthiobiotin, biocytin, and the like. As used herein, the "biotin-binding site" includes avidin and its analogs. Examples of the avidin analogs include streptavidin, Pleurotus cornucopiae-derived avidin-like protein (tamavidin (registered trademark)), bradavidin, rhizavidin, and the like.
Examples of the tagged cholesterol include biotin-added cholesterol represented by following formula (II), (III) or (IV): 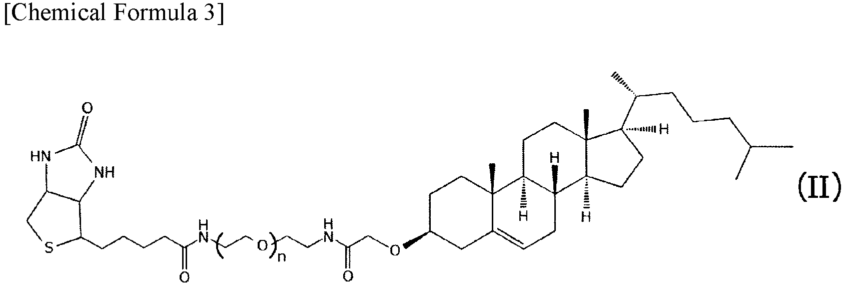 wherein n is an integer of 1 or more and 23 or less, 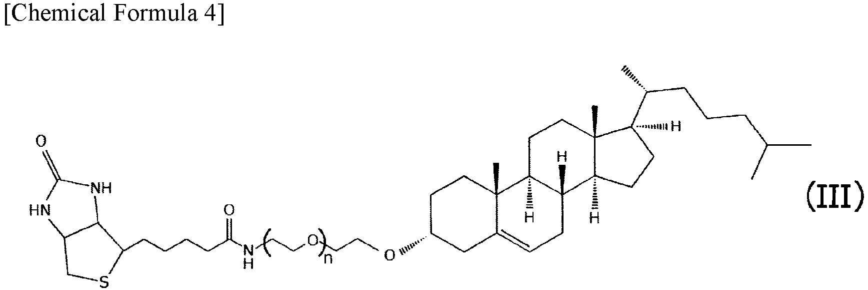 wherein n is an integer of 1 or more and 7 or less, 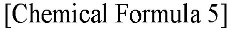 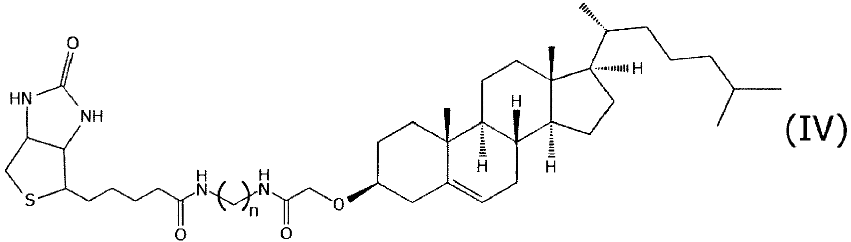 wherein n is an integer of 2 or more and 5 or less.

In the tagged cholesterol, a binding mode between the cholesterol moiety and the tag is not particularly limited, but a covalent bond is preferred. For example, a tag may be covalently bound to the C3 position of the cholesterol moiety, or the C3 position of the cholesterol moiety and the tag may be covalently bound via a linker. Although the bonding means is not particularly limited, for example, a crosslink by utilizing a functional group is preferred because it is convenient. Although the functional group is not particularly limited, for example, an amino group, a carboxyl group and a sulfhydryl group are preferred because commercially available crosslinkers can be used.

When cholesterol is used, the hydroxy group at the C3 position is poor in reactivity as a functional group. Therefore, for example, as shown in the first step in the figure below, a hydroxy group at the C3 position may be reacted with tosyl chloride to produce tosylate (in the figure, R-OH is cholesterol). Then, as shown in the second step in the figure below, by reacting tosylate with an alcohol having a tag, the tosyl group is eliminated to obtain tagged cholesterol (in the figure, R'-OH is an alcohol having a tag).

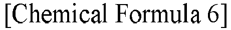

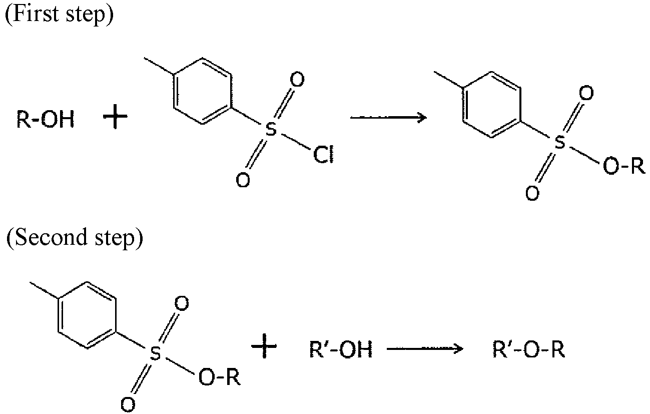

Alternatively, as shown in the first step in the figure below, a hydroxy group at the C3 position of cholesterol may be reacted with an alkylating agent such as ethyl bromoacetate, ethyl bromopropionate, or ethyl bromobutyrate to produce an ether (in the figure, R-OH is cholesterol, R' is, for example, a methylene group, an ethylene group or a propylene group, and Et is an ethyl group). Then, as shown in the second step in the figure below, a carboxy group may be imparted to the C3 position of cholesterol by hydrolyzing the product with an alkali such as potassium hydroxide. By binding a tag to the carboxy group at the C3 position by a cross-linking reaction, tagged cholesterol can be obtained.

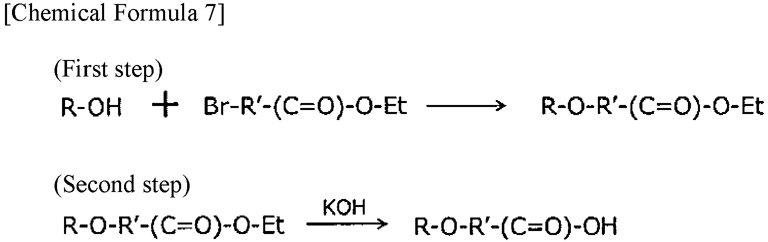

The functional group of the tag varies depending on the type of the tag. For example, when a peptide or a protein is used as the tag, an amino group, a carboxyl group and a sulfhydryl group (SH group) can be used. When biotin is used for the tag, a carboxyl group in a side chain of biotin can be used. The linker is preferably a compound having a chain structure having functional groups at both terminals (for example, a polymer compound). When biotin is added as the tag, a commercially available biotin labeling reagent may be used. The reagent contains biotin to which various length of spacer arms (for example, PEG chains) having a functional group are bound at the terminal.

Hereinafter, cross-linking reactions of representative functional groups will be described. A compound having a carboxyl group as a functional group can be bound to a compound having an amino group as a reactive group through three steps shown in the figure below. First, as shown in the first step in the figure below, a compound having a carboxyl group is reacted with a compound having a carbodiimide group (-N=C=N-) (in the figure below, 1-[3-(dimethylamino)propyl]-3-ethylcarbodiimide (WSC)). Next, as shown in the second step in the figure below, the product of the first step is reacted with NHS to form an unstable NHS ester. Then, as shown in the third step in the figure below, the product of the second step and the compound having an amino group can be crosslinked by reacting them. For example, when binding a cholesterol or linker having a carboxyl group to a tag having an amino group, they can be crosslinked in this way. When the cholesterol or linker having a carboxyl group and a tag having a carboxyl group are crosslinked, a crosslink reagent having amino groups at both ends may be used.

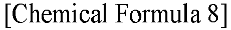

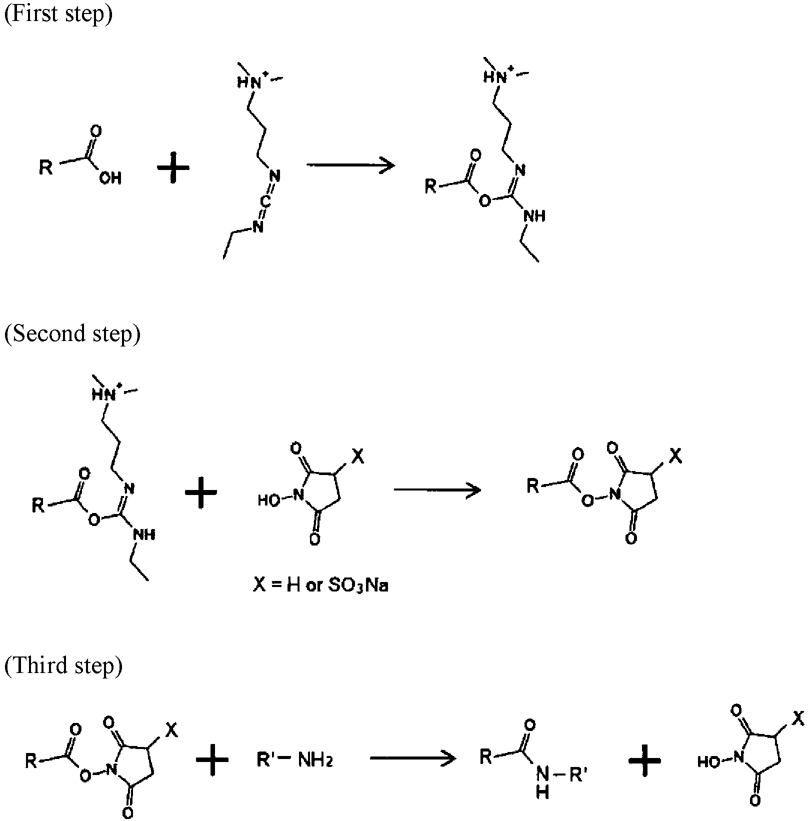

A compound having an amino group as a functional group can be crosslinked with a compound having an N-hydroxysuccinimide (NHS) ester or an isothiocyano group as a reactive group, as shown in the figure below. For example, when a linker having an amino group and a tag having an amino group are crosslinked, a crosslink reagent having NHS esters at both ends may be used.

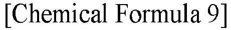

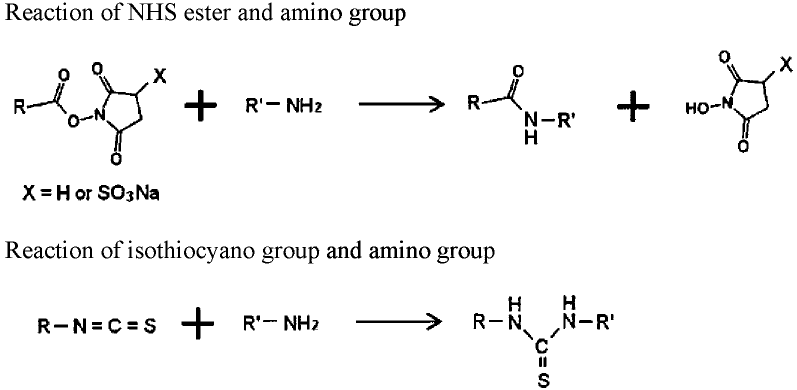

A compound having a sulfhydryl group as a functional group can be crosslinked with a compound having a maleimide group or a bromo (or iodo) acetamide group as a reactive group, as shown in the figure below. For example, when a linker having a sulfhydryl group and a tag having a sulfhydryl group are crosslinked, a crosslink reagent having maleimide at both ends may be used.

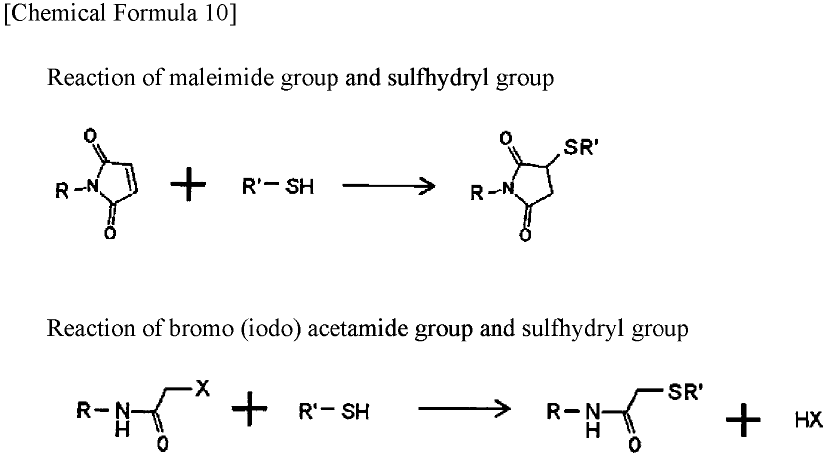

When a compound having a carboxyl group as a functional group and a compound having an amino group as a reactive group are covalently bound, an amidation reaction using a known condensing agent may be used. Examples of such a condensing agent include O-(7-azabenzotriazol-1-yl)-1,1,3,3-tetramethyluronium hexafluorophosphonate (HATU), 4-(4,6-dimethoxy-1,3,5-triazin-2-yl)-4-methylmorpholinium chloride, 2-chloro-1,3-dimethylimidazolinium, 1H-benzotriazol-1-yloxytripyrrolidinophosphonium hexafluorophosphate, diphenylphosphoryl azide, chlorotripyrrolidinophosphonium hexafluorophosphate, N,N'-diisopropylcarbodiimide, and the like.

The above cross-linking reaction and amidation reaction can be performed under normal temperature and normal pressure. The solvent used in the reaction is not particularly limited as long as it is inert to the above reaction and each compound to be subjected to the reaction can be lysed or dispersed. Examples of the solvent include aromatic hydrocarbons such as benzene, toluene and xylene, ethers such as tetrahydrofuran (THF), diethyl ether, ethylene glycol dimethyl ether and 1,4-dioxane, and amides such as N,N-dimethylformamide (DMF), sulfoxides such as dimethyl sulfoxide, halogenated hydrocarbons such as dichloromethane and chloroform, and the like. These solvents can be used alone or as a mixture.

The first capture body that specifically binds to the tag and has a labeling substance is a capture body that is labeled with the labeling substance and specifically binds to the tag. Referring to a combination of the above-mentioned tag and a substance capable of specifically binding to the tag, the substance is selected from an antibody, an aptamer, a ligand receptor, an oligonucleotide, biotin or its analog, a biotin-binding site, a histidine tag, Ni-NTA, GST, and glutathione. Among them, a biotin-binding site or an antibody is preferred. As the biotin-binding site, avidin or streptavidin is preferred.

As used herein, the term "antibody" also includes antibody fragments. Examples of the antibody fragment include Fab, Fab', F(ab')2, Fd, Fd', Fv, scFv, domain antibody (dAb), reduced IgG (rIgG), diabody, triabody, and the like. The antibody may be either a monoclonal antibody or a polyclonal antibody. The origin of the antibody is not particularly limited, and may be an antibody derived from any mammal such as a mouse, a rat, a hamster, a rabbit, a goat, a horse, or a camel. The isotype of antibody may be any of IgG, IgM, IgE, IgA and the like and is preferably IgG. The antibody that specifically binds to the tag may be a commercially available antibody or an antibody prepared by a method known in the art.

The labeling substance is not particularly limited. Examples thereof include substances which themselves generate a signal (hereinafter also referred to as "signal generating substance"), substances which catalyze the reaction of other substances to generate a signal, and the like. Examples of the signal generating substance include fluorescent substances, radioactive isotopes, and the like. Examples of the substance which catalyzes a reaction of other substances to generate a detectable signal include enzymes. The enzyme reacts with an appropriate substrate to generate signals such as light and color. Examples of the enzymes include alkaline phosphatase (ALP), peroxidase (POD), β-galactosidase, luciferase, and the like. Examples of the fluorescent substances include fluorescent dyes such as fluorescein isothiocyanate (FITC), rhodamine and Alexa Fluor (registered trademark), fluorescent proteins such as GFP, and the like. Examples of the radioactive isotopes include ¹²⁵I, ¹⁴C, ³²P, and the like. As the labeling substance, enzymes are preferred, and ALP and POD are particularly preferred.

The labeling of the capture body that specifically binds to the tag with the labeling substance can be performed by directly or indirectly binding the labeling substance to the capture body. The capture body to which the labeling substance is directly bound is, for example, a capture body to which the labeling substance is covalently bound. This can be obtained by covalently binding the labeling substance and the capture body using, for example, a commercially available labeling kit or crosslinker. When both the capture body and the labeling substance are proteins, the capture body to which the labeling substance is directly bound may be a fusion protein of the capture body and the labeling substance. This can be prepared by a genetic recombination technique known in the art.

When the labeling substance is indirectly bound to the capture body, a substance to which the labeling substance is covalently bound and that specifically binds to the capture body can be used. For example, when the capture body that specifically binds to the tag is a biotin-binding site, biotin or its analog to which a labeling substance is covalently bound (labeled biotin or its analog) can be used. The biotin-binding site usually forms tetramers and can bind four molecules of biotin or its analogs. By mixing a biotin-binding site and labeled biotin or its analog at a ratio such that one or two molecules of labeled biotin or its analog are bound to tetrameric biotin-binding site, a biotin-binding site to which labeled biotin or its analog are bound is obtained. When the capture body that specifically binds to the tag is an antibody, an antibody to which a labeling substance is covalently bound and that specifically binds to the antibody (labeled secondary antibody) can be used.

In a preferred embodiment, the first capture body is a labeled antibody, labeled avidin, or labeled streptavidin that specifically binds to the tag. As the labeled antibody, an antibody to which a labeling substance is covalently bound or an antibody to which a labeling substance is fused is preferred. As the labeled avidin and the labeled streptavidin, avidin and streptavidin to which a labeling substance is covalently bound, avidin and streptavidin to which a labeling substance is fused, and avidin and streptavidin to which one or two molecules of labeled biotins are bound are preferred.

Contact between the high-density lipoprotein in a sample, the tagged cholesterol and the first capture body can be performed by mixing the sample, the tagged cholesterol, and the first capture body. It is considered that by this mixing, the tagged cholesterol binds to the high-density lipoprotein, and the first capture body binds to the tag exposed on the outer surface of the high-density lipoprotein. As a result, a complex containing the high-density lipoprotein containing the tagged cholesterol and the first capture body is formed. The order of mixing is not particularly limited, and the sample, the tagged cholesterol and the first capture body may be mixed substantially simultaneously or sequentially mixed.

The addition amount of the tagged cholesterol is not particularly limited. For example, the tagged cholesterol can be added to the sample to a final concentration of 10 nM to 10 µM, and preferably 20 nM to 5 µM. The addition amount of the first capture body is not particularly limited, and can be appropriately set according to the types of the capture body and the labeling substance, and the like.

In a preferred embodiment, the high-density lipoprotein in the sample is first contacted with the tagged cholesterol. Thus, the tagged cholesterol binds to the high-density lipoprotein. Thereafter, the high-density lipoprotein containing the tagged cholesterol is contacted with the first capture body. As a result, the first capture body binds to the tag exposed on the outer surface of the high-density lipoprotein, and a complex containing the high-density lipoprotein containing the tagged cholesterol and the first capture body is formed. Conditions of temperature and time in mixing the sample and the tagged cholesterol are not particularly limited. For example, a mixed solution of the sample and the tagged cholesterol may be incubated at 20 to 48°C, preferably 25 to 42°C, for 1 minute to 24 hours, and preferably 10 minutes to 2 hours. During the incubation, the mixed solution may be allowed to stand, or may be stirred or shaken. Conditions of temperature and time in mixing the high-density lipoprotein containing the tagged cholesterol and the first capture body are not particularly limited, and can be appropriately determined from the above ranges.

In a preferred embodiment, a second capture body that specifically binds to the high-density lipoprotein is used for contacting the high-density lipoprotein, the tagged cholesterol, and the first capture body. Specifically, the sample, the tagged cholesterol, the first capture body, and the second capture body are mixed. The order of mixing is not particularly limited. The addition amount of the second capture body is not particularly limited, and can be appropriately set according to the type of the capture body and the like.

The second capture body is not particularly limited as long as it is a substance capable of specifically binding to a part of the surface of high-density lipoprotein. Examples of such substances include antibodies, aptamers, and the like. As the second capture body, an antibody that specifically binds to high-density lipoprotein is preferred, and an antibody that can specifically bind to apolipoprotein that is a component of high-density lipoprotein is more preferred. Examples of such antibodies include anti-ApoA antibodies (anti-ApoAI antibody and anti-ApoAII antibody), anti-ApoB antibody, anti-ApoE antibodies (anti-ApoE2 antibody, anti-ApoE3 antibody and anti-ApoE4 antibody), and the like. Among them, anti-ApoAI antibodies are particularly preferred. A commercially available anti-lipoprotein antibody or anti-ApoAI antibody may be used.

In the contact between the high-density lipoprotein, the tagged cholesterol and the first capture body, by further contacting the second capture body, a complex of the first capture body, the high-density lipoprotein to which the tagged cholesterol is bound, and the second capture body is formed. In this complex, the first capture body binds to the tag exposed on the outer surface of the high-density lipoprotein, and the second capture body binds to the surface of the high-density lipoprotein. That is, the high-density lipoprotein containing the tagged cholesterol is sandwiched between the first capture body and the second capture body. In this embodiment, the complex of the first capture body, the high-density lipoprotein containing the tagged cholesterol and the second capture body is also hereinafter referred to as the "sandwich complex".

The preferred order of contact in the formation of the sandwich complex is as follows. First, the high-density lipoprotein in the sample is contacted with the tagged cholesterol. Thus, the tagged cholesterol binds to the high-density lipoprotein. Next, the high-density lipoprotein containing the tagged cholesterol is contacted with the second capture body. As a result, the second capture body binds to the surface of the high-density lipoprotein containing the tagged cholesterol, and a complex of the high-density lipoprotein containing the tagged cholesterol and the second capture body is formed. Thereafter, the complex of the high-density lipoprotein containing the tagged cholesterol and the second capture body is contacted with the first capture body. As a result, the first capture body binds to the tag exposed on the outer surface of the high-density lipoprotein to form a sandwich complex.

There are no particular limitations on the conditions of temperature and time in the mixing of the high-density lipoprotein containing the tagged cholesterol and the second capture body. For example, a mixed solution of the high-density lipoprotein containing the tagged cholesterol and the second capture body may be incubated at 20 to 48°C, preferably 25 to 42°C, for 1 minute to 24 hours, and preferably 10 minutes to 2 hours. During the incubation, the mixed solution may be allowed to stand, or may be stirred or shaken.

The complex of the high-density lipoprotein containing the tagged cholesterol and the second capture body may be formed on a solid phase. For example, by contacting the high-density lipoprotein in the sample, the tagged cholesterol, the second capture body, and the solid phase with each other, the complex is formed on the solid phase. The order of contact is not particularly limited, but for example, after contacting the high-density lipoprotein in the sample with the tagged cholesterol, the high-density lipoprotein containing the tagged cholesterol, the second capture body, and the solid phase may be contacted with each other. Alternatively, after contacting the high-density lipoprotein containing the tagged cholesterol with the second capture body, the complex of the high-density lipoprotein containing the tagged cholesterol and the second capture body may be contacted with the solid phase.

The second capture body may be previously immobilized on the solid phase. For example, after contacting the high-density lipoprotein in the sample with the tagged cholesterol, the high-density lipoprotein containing the tagged cholesterol may be contacted with the solid phase on which the second capture body is immobilized. Alternatively, the high-density lipoprotein containing the tagged cholesterol may be contacted with the solid phase on which the second capture body is immobilized. The conditions of temperature and time when using a solid phase are not particularly limited. For example, the conditions may be the same as those for the mixing of the high-density lipoprotein containing the tagged cholesterol and the second capture body.

The solid phase may be any insoluble carrier capable of immobilizing the second capture body. For example, the capture body can be immobilized on the solid phase by directly or indirectly binding the solid phase and the second capture body. Examples of the direct binding between the solid phase and the second capture body include adsorption or covalent binding to the surface of the solid phase by hydrophobic interaction. For example, when the second capture body is an antibody and the solid phase is an ELISA microplate, the antibody can be immobilized in a well of the plate by adsorption. When the second capture body is an antibody and the solid phase has a functional group on the surface, the antibody can be immobilized on the surface of the solid phase by covalent binding using the functional group. For example, when the solid phase is a particle having a carboxyl group, the cross-linking reaction of the compound having a carboxyl group described above can be used. Specifically, a carboxyl group on the particle surface is activated with WSC and then reacted with NHS to form an NHS ester. Then, when the particle having the NHS ester is contacted with the antibody, the NHS ester reacts with an amino group of the antibody, and the antibody is covalently immobilized on the particle surface.

Examples of the indirect binding between the solid phase and the second capture body include binding via a molecule that specifically binds to the second capture body. By previously immobilizing such a molecule on the surface of the solid phase, the second capture body can be immobilized on the solid phase. For example, when the second capture body is an antibody, examples of the molecule that specifically binds to the second capture body include protein A or protein G, an antibody (a secondary antibody) that specifically recognizes an antibody, and the like. A combination of substances interposed between the second capture body and the solid phase can be used to bind them. Examples of the combination of substances include combinations of any of biotin and its analogs and any of biotin-binding sites, a hapten and an anti-hapten antibody and the like. For example, when the second capture body is previously modified with DNP, by a solid phase on which an anti-DNP antibody is immobilized, the second capture body can be immobilized on the solid phase.

The solid phase material can be selected from organic polymer compounds, inorganic compounds, biopolymers, and the like. Examples of the organic polymer compounds include latex, polystyrene, polypropylene, styrene-methacrylic acid copolymer, styrene-glycidyl (meth)acrylate copolymer, styrene-styrene sulfonate copolymer, methacrylic acid polymer, acrylic acid polymer, acrylonitrile butadiene styrene copolymer, vinyl chloride-acrylate copolymer, polyvinyl acetate acrylate, and the like. Examples of the inorganic compounds include magnetic bodies (iron oxide, chromium oxide, cobalt, ferrite, etc.), silica, alumina, glass, and the like. Examples of the biopolymer include insoluble agarose, insoluble dextran, gelatin, cellulose, and the like. Two or more of these may be used in combination.

The shape of the solid phase is not particularly limited. Examples of the shape of the solid phase include a particle, a microplate, a microtube, a test tube, and the like. Among them, a particle and a microplate are preferred, and a magnetic particle is particularly preferred. When the shape of the solid phase is a particle, a suspension of the particles can be used for forming the above complex as the solid phase. When the shape of the solid phase is a container such as a microplate, the above complex can be formed in the container as the solid phase. When the second capture body immobilized on the magnetic particle is used, measurement may be carried out using a commercially available fully automated immunoassay system such as HISCL (registered trademark) series (Sysmex Corporation).

B/F (Bound/Free) separation for removing an unreacted free component may be performed between the contact between the high-density lipoprotein containing the tagged cholesterol and the second capture body and the contact between the formed complex and the first capture body. The unreacted free component is a component that does not constitute the complex of the high-density lipoprotein containing the tagged cholesterol and the second capture body. Examples thereof include free tagged cholesterol that has not bound to high-density lipoprotein, a free second capture body that has not bound to high-density lipoprotein, impurities in the sample, and the like. The means for B/F separation is not particularly limited, but the complex and the unreacted free component can be separated by recovering only the complex by, for example, ultracentrifugation. In a case where the complex is formed on a solid phase, when the solid phase is particles, the complex and the unreacted free component can be separated by recovering the particles by centrifugation or magnetic separation, and removing the supernatant. When the solid phase is a container such as a microplate or a microtube, the complex and the unreacted free component can be separated by removing a liquid containing the unreacted free component.

After removing the unreacted free components, the recovered complex or a solid phase on which the complex is immobilized can be washed with a suitable aqueous medium. Examples of the aqueous medium include water, physiological saline, PBS and Tris-HCl, Good buffers, and the like. If necessary, a surfactant may be added to the aqueous medium. The surfactant is not particularly limited, and for example, can be appropriately selected from surfactants used for a washing buffer in the fields of biochemistry and molecular biology.

In the measurement method of the present invention, a signal generated by a labeling substance is detected. The labeling substance is a labeling substance of the first capture body that binds to the tag exposed on the outer surface of the high-density lipoprotein. Therefore, the signal generated by this labeling substance reflects the amount of tagged cholesterol in the high-density lipoprotein. That is, the detection result of the signal is an index of high-density lipoprotein's ability to uptake cholesterol.

As used herein, the phrase "detecting a signal" includes qualitatively detecting the presence or absence of a signal, quantifying a signal intensity, and semi-quantitatively detecting the signal intensity. The phrase "semi-quantitatively detecting the signal intensity" means to detect the signal intensity in plural stages such as "no signal generated", "weak", "strong", and the like. Preferably, the intensity of the signal generated by the labeling substance contained in the complex is quantified to obtain a measured value. If necessary, a value obtained by subtracting the background value from the measured value of the signal intensity may be obtained. Examples of the background value include a measured value of signal intensity obtained by measurement without using any one of the sample, the tagged cholesterol, the first capture body, and the second capture body.

Methods for detecting a signal themselves are known in the art. In the methods of the present invention, a suitable measurement method can be selected according to the type of signal derived from the labeling substance. For example, when the labeling substance is an enzyme, signals such as light and color generated by reacting an enzyme and a substrate for the enzyme can be measured by using a known apparatus. Examples of the measuring device include a spectrophotometer, a luminometer, and the like.

The substrate of the enzyme can be appropriately selected from known substrates according to the type of the enzyme. For example, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB). When alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.1^{3,7}]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate. In a preferred embodiment, the signal is a chemiluminescent signal generated by contacting the enzyme with the substrate.

When the labeling substance is a radioactive isotope, radiation as a signal can be measured using a known apparatus such as a scintillation counter. When the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used.

The B/F separation for removing an unreacted free component may be performed before signal detection. Examples of the unreacted free component include a free first capture body that has not bound to the tag, and the like. A specific method of the B/F separation and a washing solution are similar to those in the above-mentioned washing.

Each of the above-described steps is performed in vitro. Each of the above-mentioned steps is performed in a substantially cell-free system. The cell-free system substantially means that cells are not positively added for the purpose of being used to measure cholesterol in high-density lipoprotein. For example, in the conventional measurement method of cholesterol efflux function, cholesterol-accumulated cells such as macrophage are used. However, since the tagged cholesterol is directly incorporated binds to the high-density lipoprotein in the sample in the measurement method of the present invention, it is not necessary to use such cells. Even when the sample contains cells derived from a living body, it is considered that the cells themselves hardly affect binding between the high-density lipoprotein and the tagged cholesterol, and the measurement method is considered to be cell-free system.

Not according to the claimed invention is a reagent for measuring cholesterol in high-density lipoprotein (hereinafter, also referred to as "the reagent"). The reagent contains tagged cholesterol, and can be used in the measurement method of the present invention. Details of the tagged cholesterol are the same as those described for the measurement method of the present invention.

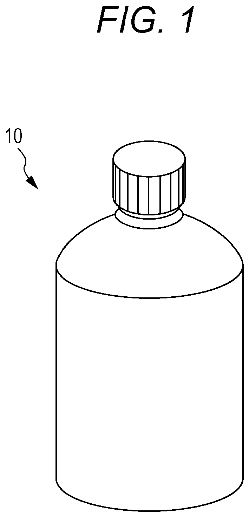

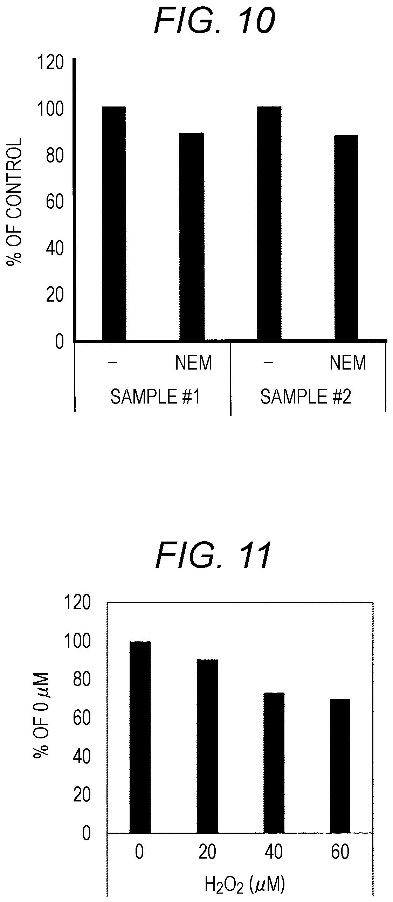

The reagent may be provided to a user by storing the tagged cholesterol in a container. Fig. 1 shows an example of the reagent. Referring to Fig. 1, 10 denotes a reagent stored in a container. The tagged cholesterol in the reagent may be solid (for example, powder, crystal, freeze-dried product, or the like) or liquid (for example, solution, suspension, emulsion, or the like). When the tagged cholesterol is contained in the reagent in the form of a liquid, examples of the solvent include the aqueous medium described above. If necessary, a stabilizer such as casein or BSA may be added to the aqueous medium.

Further disclosed herein but not according to the claimed invention is use of a tagged cholesterol for production of a reagent for measuring cholesterol in high-density lipoprotein, in which the tag is added to C3 position of a sterol skeleton in the tagged cholesterol. Details of the tagged cholesterol are the same as those described for the measurement method of the present invention.

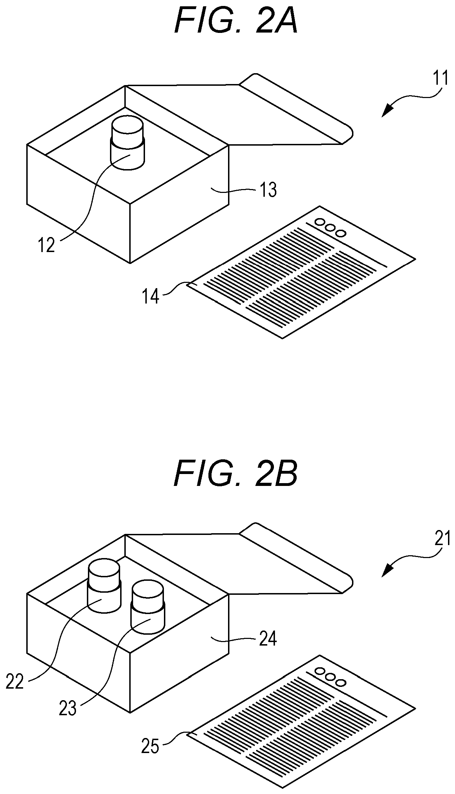

Also disclosed herein but not according to the claimed invention is a reagent kit for measuring cholesterol in high-density lipoprotein (hereinafter, also referred to as "the reagent kit"), including a reagent containing tagged cholesterol. The reagent kit can be used for the measurement method of the present invention described above. Details of the tagged cholesterol are the same as those described for the measurement method of the present invention. For example, a container containing a reagent containing tagged cholesterol may be packed in a box and provided to a user as the reagent kit. The box may contain a package insert. The package insert may describe composition of the reagent, structure of the tagged cholesterol, the method of using the reagent, the method of storing the reagent, and the like. Fig. 2A shows an example of the reagent kit. Referring to Fig. 2A, 11 denotes an example of the reagent kit, 12 denotes a container containing a reagent containing tagged cholesterol, 13 denotes a packing box, and 14 denotes a package insert.

The reagent kit may include a first reagent containing tagged cholesterol and a second reagent containing a first capture body that specifically binds to the tag and has a labeling substance. The reagent kit may further include a third reagent containing a second capture body that specifically binds to high-density lipoprotein. Details of the first capture body and the second capture body are the same as those described for the measurement method of the present invention.

The reagent kit may be provided to a user by packing a container containing each reagent in a box. The box may contain a package insert. The package insert may describe composition of each reagent, structure of the tagged sterol, the method of using each reagent, the method of storing each reagent, and the like. Fig. 2B shows an example of the reagent kit. Referring to Fig. 2B, 21 denotes an example of the reagent kit, 22 denotes a first container containing a first reagent containing tagged cholesterol, 23 denotes a second container containing a second reagent containing the first capture body, 24 denotes a packing box, and 25 denotes a package insert.

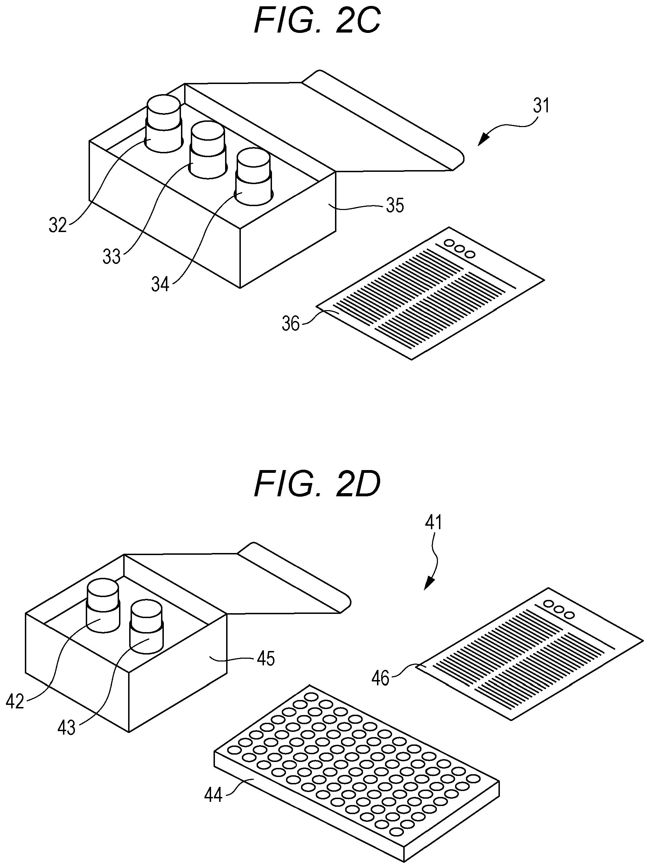

The reagent kit may include a third reagent containing a second capture body in addition to the first reagent and the second reagent. Referring to Fig. 2C, 31 denotes an example of the reagent kit, 32 denotes a first container containing a first reagent containing tagged cholesterol, 33 denotes a second container containing a second reagent containing the first capture body, 34 denotes a third container containing a third reagent containing the second capture body, 35 denotes a packing box, and 36 denotes a package insert.

Each of the tagged cholesterol in the reagent, the first capture body and the second capture body may be solid (for example, powder, crystal, freeze-dried product, or the like) or liquid (for example, solution, suspension, emulsion, or the like). When the tagged cholesterol is contained in the reagent in the form of a liquid, examples of the solvent include the aqueous medium described above. If necessary, a stabilizer such as casein or BSA may be added to the aqueous medium.

In the third reagent, the second capture body may be previously immobilized on a solid phase. In this case, the third reagent contains the second capture body immobilized on the solid phase. Details of the solid phase are the same as those described for the measurement method of the present invention. When the solid phase is a particle, in Fig. 2C, 34 denotes a container containing a reagent containing a second capture body immobilized on the particle. Fig. 2D shows an example of a reagent kit when the solid phase is a microplate and the third reagent is a microplate on which the second capture body is immobilized. Referring to Fig. 2D, 41 denotes an example of the reagent kit, 42 denotes a first container containing a first reagent containing tagged cholesterol, 43 denotes a second container containing a second reagent containing the first capture body, 44 denotes a microplate on which the second capture body is immobilized, 45 denotes a packing box, and 46 denotes a package insert. In Fig. 2D, the microplate is a 96 well plate.

The reagent kit may further include a calibrator. The calibrator includes, for example, a buffer solution containing no high-density lipoprotein (negative control) and a buffer solution containing a known concentration of high-density lipoprotein. The reagent kit may further include a washing solution. Details of the washing solution and an aqueous medium and surfactant contained therein are the same as those described for the measurement method of the present invention.

Further disclosed herein but not according to the claimed invention is use of a tagged cholesterol, a first capture body that specifically binds to the tag and has a labeling substance, and a second capture body that specifically binds to the high-density lipoprotein, for production of a reagent kit for measuring cholesterol in high-density lipoprotein, in which the tag is added to C3 position of a sterol skeleton in the tagged cholesterol. Details of the tagged cholesterol, the first capture body and the second capture body are the same as those described for the measurement method of the present invention as described herein.

Hereinbelow, the present invention will be described in detail by examples, but the present invention is not limited to these examples.

### EXAMPLES

### Example 1: Preparation of Tagged Cholesterol

As tagged cholesterols, biotin-PEGₙ-cholesterol, biotin-(CH₂)ₙ-cholesterol and biotin-PEG7 (ether)-cholesterol were prepared. All of the biotin groups in these biotin-added cholesterols were biotin groups of D-biotin. Respective structural formulae are shown below. 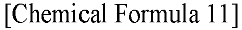 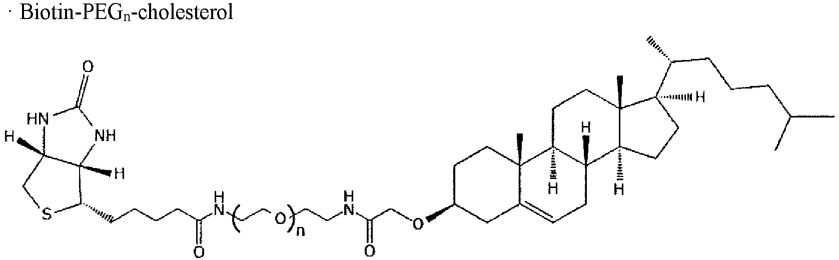 wherein n is 1, 2, 3, 7, 11, or 23. 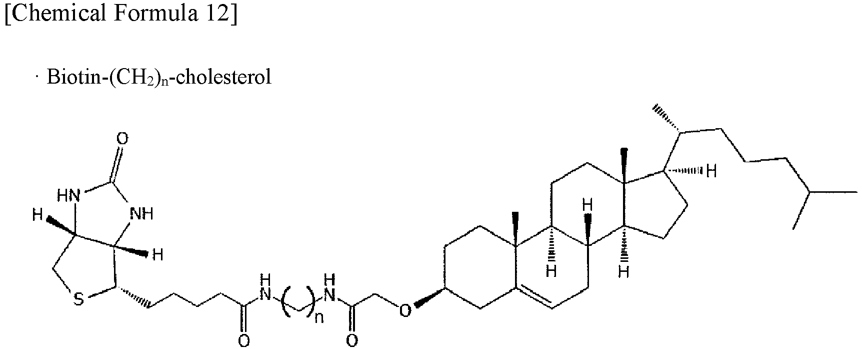 wherein n is 2 or 5.

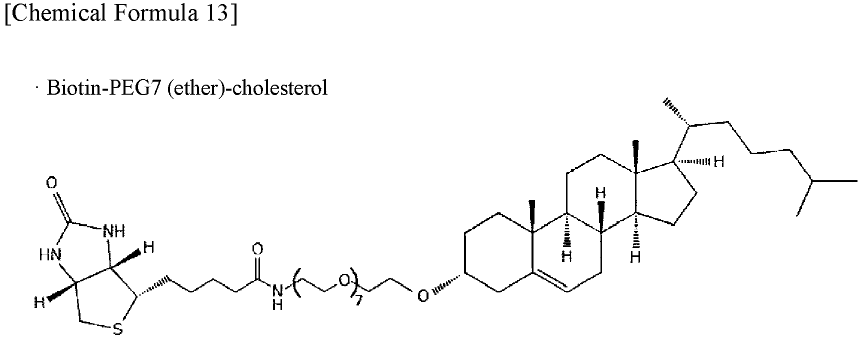

### (1) Preparation of biotin-PEGₙ-cholesterol

Synthesis scheme of biotin-PEGₙ-cholesterol is shown below.

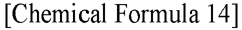

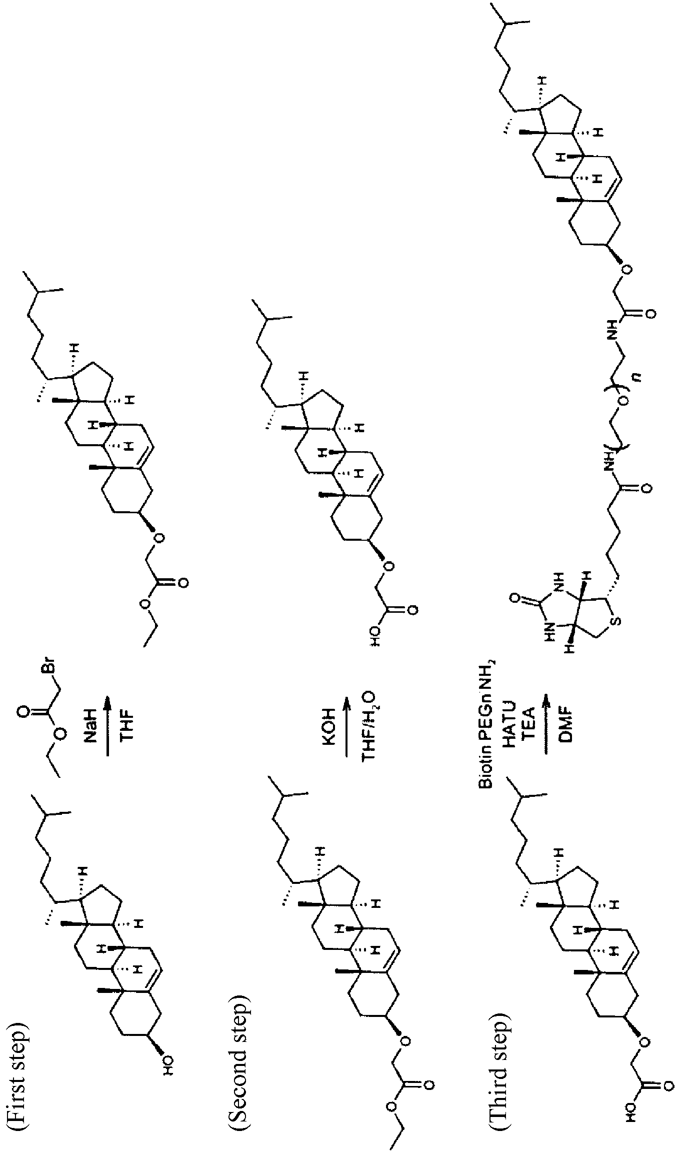

Referring to a first step, cholesterol (3 mmol) (Tokyo Chemical Industry Co., Ltd. (hereinafter, also referred to as TCI)), ethyl bromoacetate (6 mmol), and sodium hydride (9 mmol) were dissolved in DMF (15 mL), and the solution was stirred at room temperature under an argon atmosphere for 18 hours, and then purified by a silica gel column chromatography. Referring to a second step, the compound (0.11 mmol) obtained in the first step was dissolved in a mixed solution of 2.5 mL of THF and 0.5 mL of water. Subsequently, ground potassium hydroxide (0.22 mmol) was added thereto, and the mixture was stirred at room temperature under an argon atmosphere for 1 hour. A solution of the starting material (cholesterol), the reaction liquid obtained in the first step, and the reaction liquid obtained in the second step were each spotted on a silica gel plate, and the spots were developed with a developing solvent (hexane : ethyl acetate = 10 : 1). The starting material had an Rf value of 0, the product of the first step had an Rf value of 0.3, and the product of the second step had an Rf value of 0.

Referring to a third step, the compound (0.03 mmol) obtained in the second step, Biotin-PEGₙ-Amine (0.03 mmol, n is 1, 2, 3, 7, 11 or 23) (BroadPharm), HATU (0.05 mmol), and triethylamine (TEA) (8.3 µL) were dissolved in DMF (1 mL), and the mixture was stirred at room temperature under an argon atmosphere for 18 hours. The reaction liquid obtained in the second step, the reaction liquid obtained in the third step, and a mixture thereof were each spotted on a silica gel plate, and the spots were developed with a developing solvent (dichloromethane : methanol = 10 : 1). The product of the third step (Biotin-PEGₙ-cholesterol) had an Rf value of 0.1 to 0.2. Biotin-PEGₙ-cholesterol was obtained as tagged cholesterol by preparative high performance liquid chromatography. Hereinafter, when referring to a predetermined biotin-PEGₙ-cholesterol, it is referred to as "PEG1", "PEG2", "PEG3", "PEG7", "PEG11", or "PEG23" depending on the length of the PEG chain (the number of n).

### (2) Preparation of biotin-(CH₂)ₙ-cholesterol

Biotin-(CH₂)ₙ-cholesterol was prepared in the same manner as in the above (1) except that N-(2-aminoethyl)biotinamide (0.03 mmol) (Cat No. A3131: TCI) or biotin-C5-amine (0.03 mmol) (Cat No. A3155: TCI) was used instead of Biotin-PEGₙ-Amine in the third step of the above (1). Hereinafter, when referring to a predetermined biotin-(CH₂)ₙ-cholesterol, it is referred to as "C2-Amide" or "C5-Amide" depending on the number of carbon atoms (the number of n) between nitrogen atoms of the two amide groups.

### (3) Preparation of biotin-PEG7 (ether)-cholesterol

A synthesis scheme of biotin-PEG7 (ether)-cholesterol is shown below.

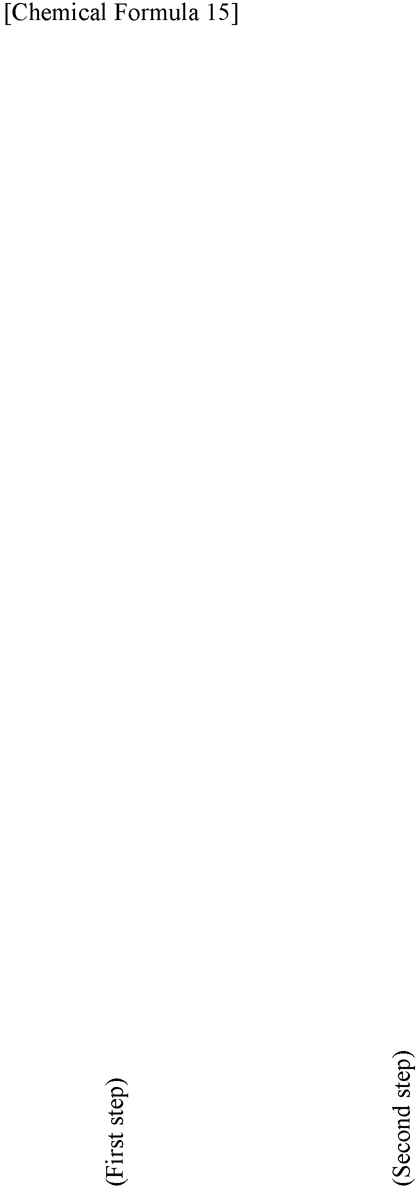

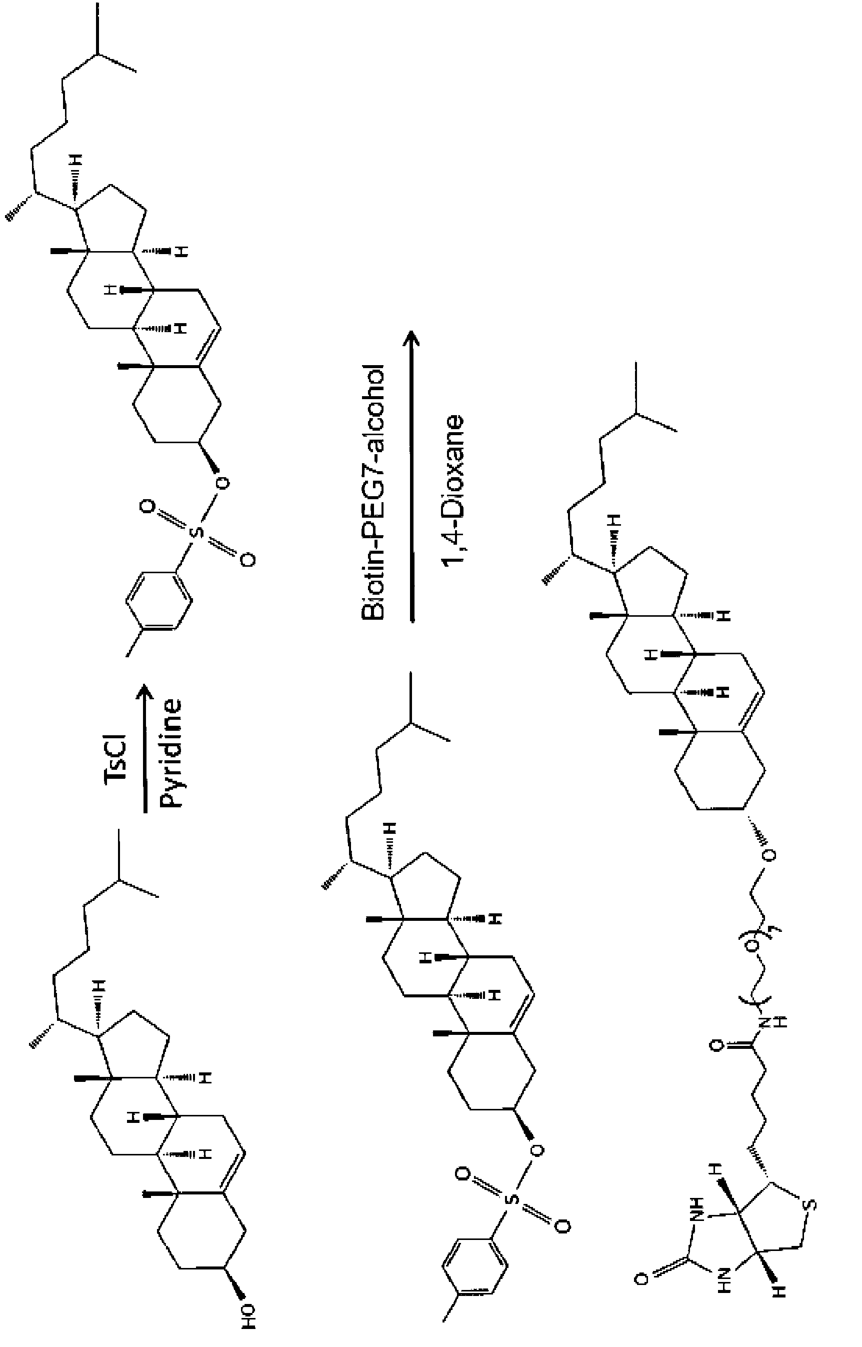

Cholesterol (1.94 mmol) (TCI) was disolved in pyridine (6 mL) , and the resulting solution was cooled to 0°C. Tosyl chloride (3.87 mmol) was dissolved in pyridine (1.2 mL). Referring to the first step, the tosyl chloride solution was added to the cholesterol solution, and the mixed solution was stirred at room temperature overnight. The reaction solution obtained in the first step was concentrated by a rotary evaporator, and the obtained solid was dissolved in 1.2 mL of chloroform and recovered. Methanol (15 mL) was added thereto to obtain a precipitate of cholest-5-en-3β-tosylate. A precipitate was obtained by filtration, washed with methanol (15 mL) and acetonitrile (6 mL), and dried. Referring to the second step, cholest-5-en-3β-tosylate (0.02 mmol) obtained in the first step and Biotin-PEG7-alcohol (BroadPharm) were dissolved in 0.2 mL of 1,4-dioxane, and the solution was heated under reflux at 110°C for 18 hours. Biotin-PEG7 (ether)-cholesterol (hereinafter, also referred to as "PEG7 (ether)") was recovered as tagged cholesterol by a silica gel column chromatography. As shown in the above synthesis scheme, in PEG7 (ether), a tosyl group is eliminated by a nucleophilic reaction with Biotin-PEG7-alcohol, and a tag is added to the oxygen atom at 3-position of a sterol skeleton, so that it is considered that the configuration of the oxygen atom at 3-position is inverted.

### Example 2: Measurement of cholesterol in high-density lipoprotein (1)

Among the biotin-added cholesterols prepared in Example 1, C5-Amide, PEG3, PEG7, PEG11 and PEG23 were used. Each biotin-added cholesterol was mixed with a sample containing high-density lipoprotein at various concentrations, and then the biotin-added cholesterols in the lipoprotein were measured to examine quantitatively. In addition, two types of high-density lipoproteins with different activities were measured in the same manner to examine whether a result reflecting qualitative activity of high-density lipoproteins was obtained. The measurement was performed with an automated high-sensitive immunoassay system for research applications HI-1000 (Sysmex Corporation).

### (1) Preparation of samples

### (1.1) Preparation of samples with different high-density lipoprotein concentrations

Pooled serum of a healthy subject was mixed with an equal amount of 22% polyethylene glycol 4000 (Nacalai Tesque, Inc.), and the mixture was allowed to stand at room temperature for 20 minutes. Thereafter, the mixed liquid was centrifuged at 860 × g at room temperature for 15 minutes. The resulting supernatant was collected as an HDL fraction. The obtained HDL fraction was diluted stepwise with PBS to prepare five samples with different HDL concentrations. Hereinafter, these samples are referred to as "C1","C2", "C3","C4", and "C5" in ascending order of HDL concentration. PBS was used as a calibrator "C0" not containing an HDL fraction.

### (1.2) Preparation of sample containing HDL with different abilities to uptake cholesterol

Each of two types of pooled sera known to have different HDL's abilities to uptake cholesterol was treated in the same manner as in the above (1.1) to collect HDL fractions. Each HDL fraction was diluted with PBS at the same magnification. Hereinafter, the diluted HDL fraction derived from the serum containing HDL having a high uptake ability is referred to as "Sample H", and the diluted HDL fraction derived from the serum containing HDL having a low uptake ability is referred to as "Sample L". The HDL's ability to uptake cholesterol in the pooled serum was determined by the method described in U.S. Patent Application Publication No. 2017/0315112.

### (2) Preparation of reagents

### (2.1) R1 Reagent (reagent containing tagged sterol)

As an R1 reagent, a buffer solution containing each biotin-added cholesterol and 0.3% sodium caseinate was prepared. The concentration of biotin-added cholesterol in each R1 reagent was 0.5 µM.

### (2.2) R2 Reagent (reagent containing solid phase on which second capture body is immobilized)

As an R2 reagent, a magnetic particle on which anti-ApoAI antibody was immobilized was prepared. Specifically, the R2 reagent was prepared as follows. WSC (DOJINDO LABORATORIES) and NHS (Kishida Chemical Co., Ltd.) were added to a magnetic particle, and then an anti-ApoA1 antibody was added to bind the anti-ApoA1 antibody to a carboxy group on a surface of the magnetic particle. As a result, a suspension containing a magnetic particle on which the anti-ApoAI antibody was immobilized was obtained as the R2 reagent.

### (2.3) R3 Reagent (reagent containing first capture body)

A buffer solution containing an alkaline phosphatase (ALP)-labeled streptavidin solution (Promega Corporation) was used as an R3 reagent.

### (2.4) R4 Reagent (measurement buffer) and R5 reagent (substrate solution)

As an R4 reagent and an R5 reagent, HISCL (registered trademark) luminescent substrate set (Sysmex Corporation) including a measurement buffer and a chemiluminescent substrate solution of ALP was used.

### (2.5) Washing solution

As a washing solution, Kolliphor (registered trademark) P188 (Sigma Aldrich) as a surfactant was dissolved in a buffer solution to prepare a buffer solution containing 0.1% (w/v) Kolliphor P188.

### (3) Measurement

### (3.1) Contact of HDL with tagged cholesterol

Each of the R1 to R5 reagents was set in HI-1000 (Sysmex Corporation). As samples, calibrators C0 to C5, Sample H, and Sample L were used. Each sample (30 µL) was added to and mixed with the R1 reagent (90 µL), and the mixture was allowed to stand at 42°C for 3 minutes. Thus, the HDL was contacted with the biotin-added cholesterol to bind the HDL to the biotin-added cholesterol.

### (3.2) Formation of complex of HDL and anti-ApoA1 antibody on magnetic particle

The R2 reagent (30 µL) was added to a cuvette containing a mixed liquid (120 µL) of the R1 reagent and the sample, and the cuvette was allowed to stand at 42°C for 2 minutes. Thus, HDL was reacted with the anti-ApoA1 antibody. Thereafter, the magnetic particles in the mixed solution were magnetically collected, the supernatant was removed, and the magnetic particles were washed using a washing solution. The magnetic particles were magnetically collected, the supernatant was removed, and then the R3 reagent (100 µL) was added to the magnetic particles on which the complex of HDL to which biotin-added cholesterol was bound and the anti-ApoA1 antibody was immobilized, and the magnetic particles were allowed to stand at 42°C for 3 minutes. As a result, the biotin-added cholesterol bound to HDL was contacted with the ALP-labeled streptavidin. The magnetic particles in the mixed liquid were magnetically collected, the supernatant was removed, and a washing liquid was added to wash the magnetic particles.

### (3.3) Measurement of tagged cholesterol in HDL

The washed magnetic particles were magnetically collected, and the supernatant was removed. The R4 reagent (50 µL) and the R5 reagent (100 µL) were added to a cuvette containing magnetic particles, and the mixture was reacted at 37°C for 5 minutes. After the reaction, chemiluminescence intensity (Count) was measured.

### (4) Results

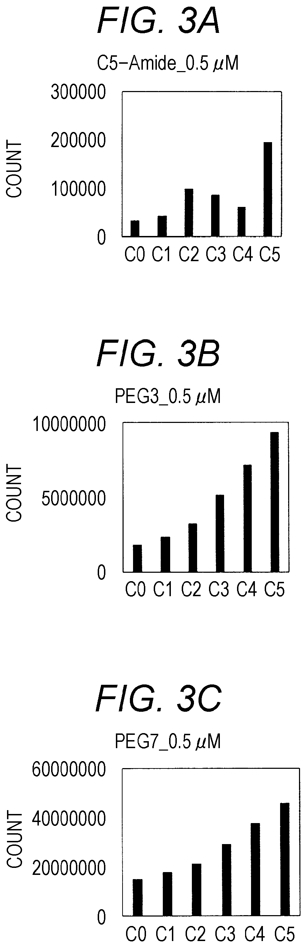

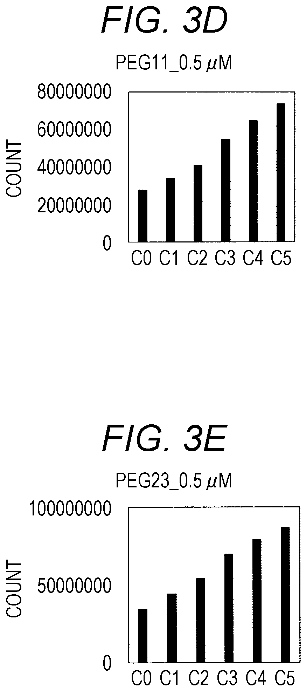

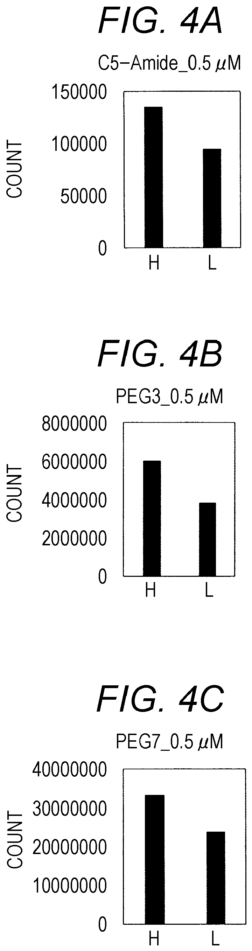

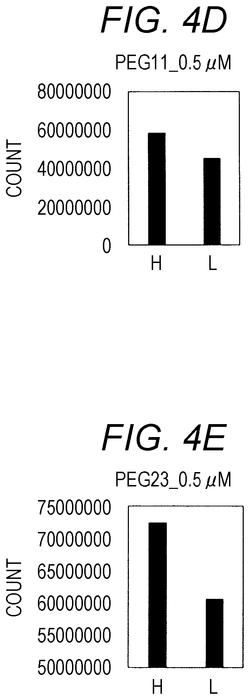

The measurement results of the calibrators are shown in Figs. 3A to 3E. The measurement results of Sample H and Sample L are shown in Figs. 4A to 4E. Referring to Figs. 3A to 3E, when any tagged cholesterol was used, the measured values of C1 to C5 were higher than the measured value of C0 containing no HDL. This showed that a signal derived from the tagged cholesterol bound to HDL could be detected. Therefore, it was shown that the tagged cholesterol in HDL can be measured using any tagged cholesterol. It was shown that the higher the HDL concentration in the calibrator, the higher the measured value. Since C1 to C5 are prepared from the same pooled serum, it is considered that more tagged cholesterols are contained in HDL in the calibrator with a high HDL concentration. Therefore, it was suggested that quantitative measurement of tagged cholesterol in HDL was possible.

When comparing the results of Figs. 3A to 3E, it was found that the detected signal intensity was higher when using tagged cholesterol with a longer linker. Fig. 3A showed that even when a spacer moiety in the linker is not PEG but a hydrocarbon chain, the tagged cholesterol in HDL can be measured.

Referring to Figs. 4A to 4E, when any tagged cholesterol was used, the measured value of Sample H was higher than the measured value of Sample L. Since Sample L and Sample H are considered to contain the HDL fraction at almost the same concentration, it was suggested that the difference in the measured values between Sample L and Sample H corresponds to the difference in the HDL's abilities to uptake cholesterol. Therefore, it was suggested that the result reflecting qualitative activity of lipoproteins is obtained by measuring the tagged cholesterol in HDL.

### Example 3: Measurement of cholesterol in high-density lipoprotein (2)

Among the biotin-added cholesterols prepared in Example 1, C2-Amide, PEG3, PEG7, PEG11, and PEG23 were used. PEG7, PEG11, and PEG23 were used at concentrations different from those in Example 2. Using each biotin-added cholesterol and Sample L and Sample H prepared in Example 2, whether the result reflecting the high-density lipoprotein activity was obtained was examined. The measurement was performed in the same manner as in Example 2.

### (1) Reagents, samples and measurements

As an R1 reagent, a buffer solution containing each biotin-added cholesterol and 0.3% sodium caseinate was prepared. The concentration of biotin-added cholesterol in each R1 reagent was 6.2 µM for C2-Amide, 0.5 µM for PEG3, 0.2 µM for PEG7, 0.1 µM for PEG11, and 0.05 µM for PEG23. As each of R2 to R5 reagents, the same reagents as in Example 2 were used. Sample L and Sample H prepared in Example 2 were used as samples. Measurement was performed in the same manner as in Example 2 except that the R1 reagent was used.

### (2) Results

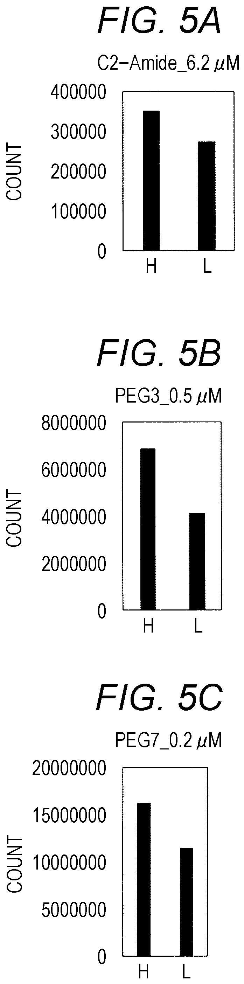

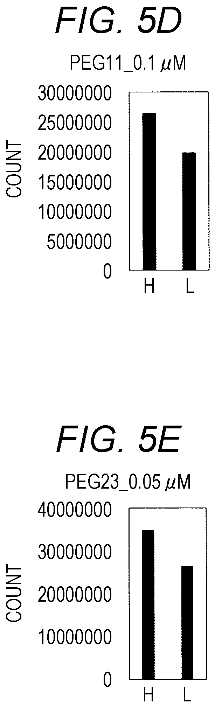

The measurement results are shown in Figs. 5A to 5E. From Figs. 5A to 5E, also in the case of using C2-Amide, the measured value of Sample H was higher than the measured value of Sample L, as in the case of using other biotin-added cholesterol. Therefore, it was suggested that even when the spacer moiety in the linker is a hydrocarbon chain with a short chain length, it is possible to measure the tagged cholesterol in HDL, and the result reflecting qualitative activity of high-density lipoproteins is obtained. Referring to Figs. 5C to 5E, the chemiluminescence intensity (count) was decreased by decreasing the concentrations of PEG7, PEG11 and PEG23 in the R1 reagent, but the results were similar to Example 2.

### Example 4: Measurement of cholesterol in high-density lipoprotein (3)

Among the biotin-added cholesterols prepared in Example 1, PEG1 and PEG2 were used. The biotin-added cholesterols in the high-density lipoprotein were measured using each biotin-added cholesterol, and the calibrators, Sample L and Sample H prepared in Example 2. The measurement was performed in the same manner as in Example 2.

### (1) Reagents, samples and measurements

As an R1 reagent, a buffer solution containing each biotin-added cholesterol and 0.3% sodium caseinate was prepared. The concentration of biotin-added cholesterol in each R1 reagent was 0.5 µM. As each of R2 to R5 reagents, the same reagents as in Example 2 were used. The calibrators C0 to C5, Sample L, and Sample H prepared in Example 2 were used as samples. Measurement was performed in the same manner as in Example 2 except that the R1 reagent was used.

### (2) Results

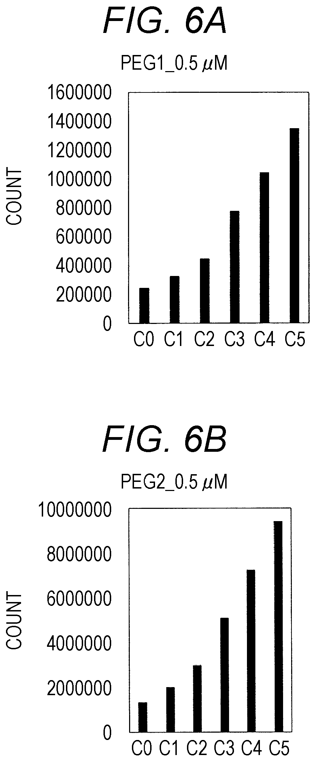

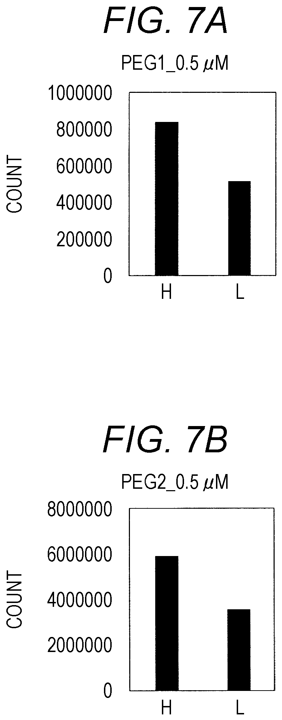

The measurement results of the calibrators are shown in Figs. 6A and 6B. The measurement results of Sample H and Sample L are shown in Figs. 7A and 7B. Referring to Figs. 6A and 6B, the measured values of C1 to C5 were higher than the measured value of C0 containing no HDL in both cases of using PEG1 and PEG2. It was shown that the higher the HDL concentration in the calibrator, the higher the measured value. Referring to Figs. 7A and 7B, the measured value of Sample H was higher than the measured value of Sample L in both cases of using PEG1 and PEG2. Therefore, it was suggested that even when tagged cholesterol whose spacer moiety in the linker is a short PEG chain is used, it is possible to quantitatively measure the tagged cholesterol in HDL, and the result reflecting qualitative activity of high-density lipoproteins is obtained.

### Example 5: Measurement of cholesterol in high-density lipoprotein (4)

Among the biotin-added cholesterols prepared in Example 1, PEG7 (ether) was used. The biotin-added cholesterol in the lipoprotein was measured using PEG7 (ether), and the calibrators, Sample L and Sample H prepared in Example 2. The measurement was performed in the same manner as in Example 2.

### (1) Reagents, samples and measurements

As an R1 reagent, a buffer solution containing 1 µM PEG7 (ether) and 0.3% sodium caseinate was prepared. As each of R2 to R5 reagents, the same reagents as in Example 2 were used. The calibrators C0 to C5, Sample L, and Sample H prepared in Example 2 were used as samples. Measurement was performed in the same manner as in Example 2 except that the R1 reagent was used.

### (2) Results

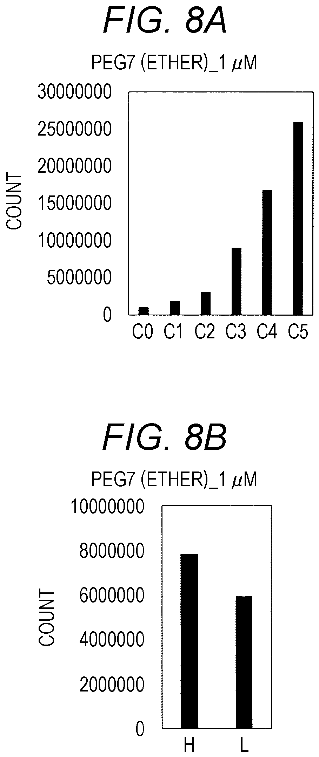

The measurement results of the calibrators are shown in Fig. 8A. The measurement results of Sample H and Sample L are shown in Fig. 8B. Referring to Fig. 8A, the measured values of C1 to C5 were higher than the measured value of C0 containing no HDL. It was shown that the higher the HDL concentration in the calibrator, the higher the measured value. Referring to Fig. 8B, the measured value of Sample H was higher than the measured value of Sample L. As described above, in PEG7 (ether), unlike other tagged cholesterols, the configuration of the oxygen atom at C3 position to which the tag was bound was inverted, but it was found that this point did not affect the measurement. Therefore, it was suggested that even when tagged cholesterol in which the connecting moiety between the C3 position of a sterol skeleton and a linker is an ether bond is used, the tagged cholesterol in HDL can be quantitatively measured, and the result reflecting qualitative activity of high-density lipoproteins is obtained.

### Example 6: Examination on effect of LCAT on cholesterol measurement (1)

The hydroxy group at the C3 position of cholesterol is esterified by LCAT and incorporated into the center of lipoprotein. However, the tagged cholesterol used in the present invention does not have the hydroxy group. Whether or not measurement of tagged cholesterol in high-density lipoprotein was affected by LCAT was examined using recombinant LCAT.

### (1) Preparation of samples

Pooled serum of a healthy subject was mixed with an equal amount of 22% polyethylene glycol 4000 (Nacalai Tesque, Inc.), and the mixture was allowed to stand at room temperature for 20 minutes. Thereafter, the mixed liquid was centrifuged at 860 × g at room temperature for 15 minutes. The resulting supernatant was collected as an HDL fraction. A portion was taken from the obtained HDL fraction, and ApoAI concentration was measured using a kit for ApoAI measurement, N-assay TIA ApoAI-H (Nittobo Medical Co., Ltd.). Specific operations for measuring the concentration were performed according to a manual attached to the kit. The recombinant LCAT (Sino Biological) was mixed with the HDL fraction so as to have a weight ratio of 1 : 10 with respect to ApoA1 contained in the HDL fraction to prepare an LCAT-added sample. For comparison, the HDL fraction to which LCAT was not added was used as an LCAT-unadded sample for measurement.

### (2) Reagents and measurement

As an R1 reagent, a buffer solution containing each biotin-added cholesterol of C2-Amide, PEG3, PEG7, PEG11, and PEG23 and 0.3% sodium caseinate was prepared. The concentration of biotin-added cholesterol in each R1 reagent was 6.2 µM for C2-Amide, 0.5 µM for PEG3, 0.2 µM for PEG7, 0.1 µM for PEG11, and 0.05 µM for PEG23. As each of R2 to R5 reagents, the same reagents as in Example 2 were used. Measurement was performed in the same manner as in Example 2 except that the above samples and R1 reagent were used. In addition, for background measurement, PBS was measured in the same manner as each sample.

### (3) Results

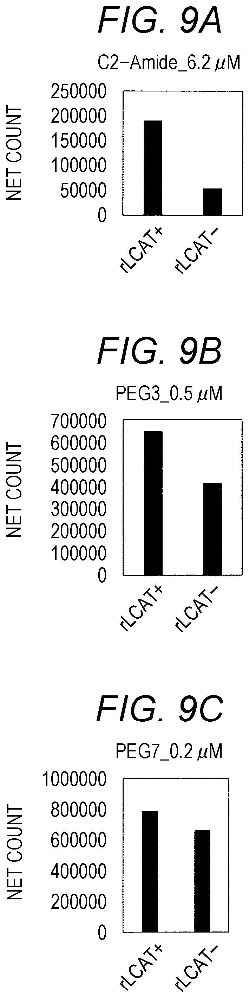

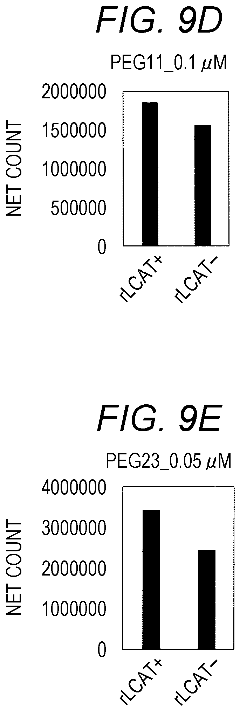

The measurement results are shown in Figs. 9A to 9E. In the figure, "Net count" refers to a value obtained by subtracting the measured value of PBS from the measured value of each sample, "rLCAT+" refers to an LCAT-added sample, and "rLCAT-" refers to an LCAT-unadded sample. Referring to Figs. 9A to 9E, when any tagged cholesterol was used, the measured value of rLCAT+ was higher than the measured value of rLCAT-. This showed that the addition of recombinant LCAT increased the signal derived from the tagged cholesterol bound to HDL. It is considered that in the presence of recombinant LCAT, esterification of endogenous cholesterol present near the surface layer of HDL and movement of endogenous cholesterol into the HDL were promoted, and the binding amount of tagged cholesterol in the surface layer of the HDL increased. Therefore, it was suggested that the measurement of tagged cholesterol in HDL is indirectly affected by LCAT.

### Example 7: Examination on effect of LCAT on cholesterol measurement (2)

Whether or not the measurement of tagged cholesterol in high-density lipoprotein was affected by LCAT was examined using an LCAT inhibitor.

### (1) Preparation of samples

N-Ethylmaleimide (NEM) as an LCAT inhibitor was added to pooled serum of a healthy subject to a final concentration of 10 mM, and the mixture was incubated at 37°C for 45 minutes. Thereafter, the pooled serum was diluted 1600-fold with PBS to prepare an NEM-added sample. For comparison, the diluted pooled serum to which NEM was not added was used as an NEM-unadded sample for measurement.

### (2) Reagents and measurement

As an R1 reagent, a buffer solution containing 0.5 µM PEG3 and 0.3% sodium caseinate was prepared. As each of R2 to R5 reagents, the same reagents as in Example 2 were used. Measurement was performed in the same manner as in Example 2 except that the above samples and R1 reagent were used. The measurement was performed twice independently.

### (3) Results

The measurement result is shown in Fig. 10. In the figure, "% of control" indicates a ratio of the measured value of the NEM-added sample when the measured value of the NEM-unadded sample is taken as 100%. Referring to Fig. 10, the measured value of the NEM-added sample was lower than the measured value of the NEM-unadded sample. This showed that the addition of NEM reduced the signal derived from the tagged cholesterol bound to HDL. It is considered that since the activity of endogenous LCAT was inhibited by NEM, the esterification of endogenous cholesterol by HDL and the movement of endogenous cholesterol into the HDL were suppressed, and the binding amount of tagged cholesterol in the HDL was reduced. Also in consideration of the results of Example 6, it was suggested that the method of the present invention can evaluate the qualitative activity of high-density lipoproteins reflecting the activity of LCAT.

### Example 8: Evaluation of qualitative activity of high-density lipoproteins by cholesterol measurement

A sample containing lhigh-density ipoprotein whose function was reduced by oxidation treatment was measured using tagged cholesterol to examine whether or not qualitative activity of the high-density lipoproteins could be evaluated.

### (1) Preparation of samples

The healthy subject serum was diluted 100-fold with PBS. Diethylenetriaminepentaacetic acid (final concentration: 100 µM, Tokyo Chemical Industry Co., Ltd.), hydrogen peroxide (final concentration: 20, 40 or 60 µM, FUJIFILM Wako Pure Chemical Corporation), recombinant myeloperoxidase (final concentration: 10 nM, R&D Systems, Inc.), and sodium nitrite (200 µM, FUJIFILM Wako Pure Chemical Corporation) were added to the diluted serum, and the mixture was incubated at 37°C for 1 hour. Thereafter, L-methionine was added so as to have a final concentration of 2 mM to stop the oxidation reaction. The obtained reaction solution was diluted 15-fold with PBS to prepare an oxidation-treated sample. For comparison, a sample prepared in the same manner as described above except that hydrogen peroxide was not added was also measured.

### (2) Reagents and measurement

As an R1 reagent, a buffer solution containing 0.5 µM PEG3 and 0.3% sodium caseinate was prepared. As each of R2 to R5 reagents, the same reagents as in Example 2 were used. Measurement was performed in the same manner as in Example 2 except that the above samples and R1 reagent were used.

### (3) Results

The measurement result is shown in Fig. 11. In the figure, "% of 0 µM" indicates a ratio of the measured value of each oxidation-treated sample when the measured value of the sample to which hydrogen peroxide was not added was taken as 100%. Referring to Fig. 11, the measured value of the oxidation-treated sample was lower than the measured value of the sample to which hydrogen peroxide was not added. It was shown that the higher the concentration of the added hydrogen peroxide, the lower the measured value. It is considered that the decrease in the activity of HDL by the oxidation treatment suppressed uptake of endogenous cholesterol by HDL, and as a result, the binding amount of tagged cholesterol in the HDL decreased.

It has been demonstrated that the method of the present invention can evaluate the qualitative activity of high-density lipoproteins.

## Claims

1. A method for measuring sterol in lipoprotein, comprising:
forming a complex by contacting with each other: lipoprotein in a sample; a tagged sterol; and a first capture body that specifically binds to the tag and comprises a labeling substance,
the complex comprising: the lipoprotein comprising the tagged sterol; and the first capture body; and
detecting a signal generated by the labeling substance comprised in the complex,
wherein in the tagged sterol, the tag is added to C3 position of a sterol skeleton, wherein the tagged sterol is tagged cholesterol and wherein the lipoprotein is high-density lipoprotein, and wherein the tag and the first capture body are a combination selected from an antigen and an antibody that recognizes the antigen, a hapten and an anti-hapten antibody, a peptide or a protein and an aptamer that recognizes them, a ligand and a receptor thereof, an oligonucleotide and an oligonucleotide having a complementary strand thereof, biotin or a biotin analog and a biotin-binding site, a histidine tag and nitrilotriacetic acid that forms a chelate with a nickel ion (Ni-NTA), and glutathione-S-transferase (GST) and glutathione.

2. The method according to claim 1, wherein
the tagged cholesterol is represented by following formula (I): 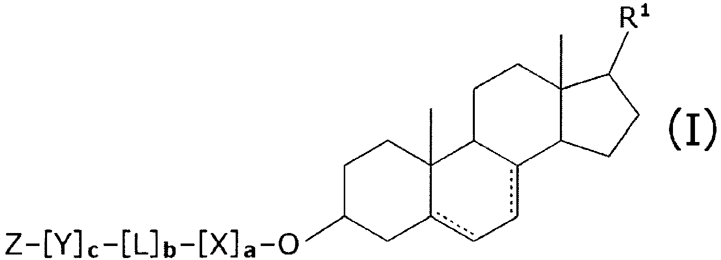
wherein double lines of a solid line and a broken line each independently represent a single bond or a double bond;
R¹ is an alkyl group having 1 or more and 6 or less carbon atoms which may have a substituent, or an alkenyl group having 2 or more and 6 or less carbon atoms which may have a substituent;
X and Y are identical or different, and are represented by -R²-NH-, -NH-R²-, -R²-(C=O)-NH-, -(C=O)-NH-R²-, -R²-NH-(C=O)-, -NH-(C=O)-R²-, -R²-(C=O)-, -(C=O)-R²-, - R²-(C=O)-O-, -(C=O)-O-R²-, -R²-O-(C=O)-, -O-(C=O)-R²-, -R²-(C=S)-NH-, -(C=S)-NH-R²-, -R²-NH-(C=S)-, -NH-(C=S)-R²-, -R²-O-, -O-R²-, -R²-S-, or -S-R²-, and each R² is independently an atomic bonding, an alkylene group having 1 or more and 10 or less carbon atoms which may have a substituent, an arylene group or heteroarylene group having 6 or more and 12 or less carbon atoms which may have a substituent, or a cycloalkylene group or heterocycloalkylene group having 3 or more and 8 or less carbon atoms which may have a substituent;
L is represented by -(CH₂)_{d}-[R³-(CH₂)ₑ]_{f}- or -[(CH₂)ₑ-R³]_{f}-(CH₂)_{d}-, and R³ is an oxygen atom, a sulfur atom, -NH-, -NH-(C=O)-, -(C=O)-NH-, or an atomic bonding;
Z is a tag;
a and c are identical or different and are an integer of 0 or more and 6 or less;
b is 0 or 1;
d and e are identical or different and are an integer of 0 or more and 12 or less; and
f is an integer of 0 or more and 24 or less.

3. The method according to claim 2, wherein in the formula (I), a is 0 or 1, b and c are 1, X is represented by -NH-(C=O)-R²-, Y is represented by -R²-(C=O)-NH-, R² is each independently an alkylene group having 1 or more and 6 or less carbon atoms and having no substituent, L is represented by -[(CH₂)₂-O]_{f}-(CH₂)_{d}-, d is an integer of 1 or more and 6 or less, and f is an integer of 0 or more and 24 or less.

4. The method according to any one of claims 1 to 3, wherein the tag is a biotin group.

5. The method according to any one of claims 1 to 4, wherein the tagged cholesterol is represented by following formula (II): 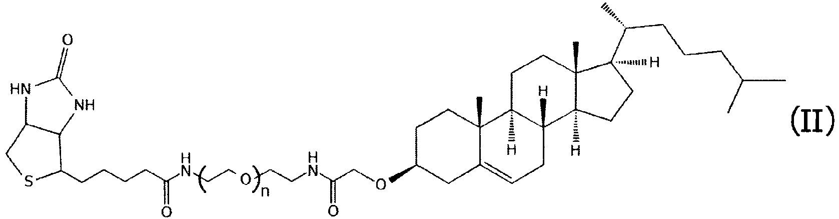 wherein n is an integer of 1 or more and 23 or less,
or following formula (III): 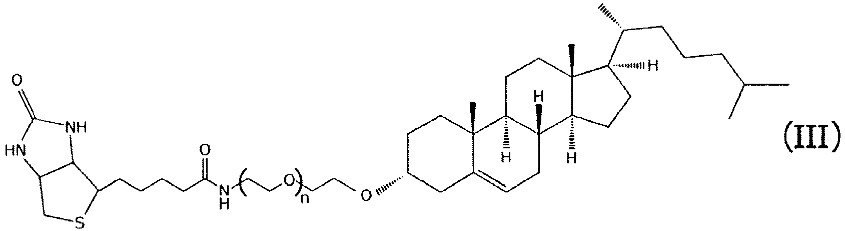 wherein n is an integer of 1 or more and 7 or less,
or following formula (IV): 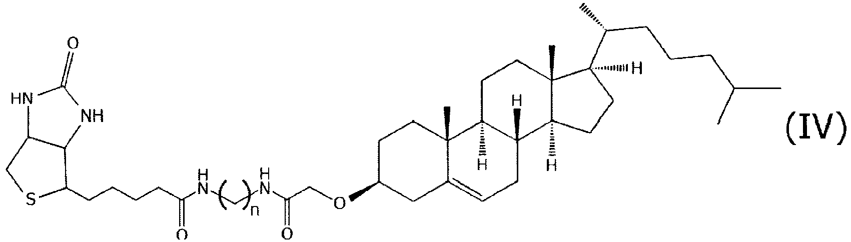 wherein n is an integer of 2 or more and 5 or less.

6. The method according to any one of claims 1 to 5, wherein the first capture body is a labeled antibody, labeled avidin, or labeled streptavidin, the first capture body specifically binding to the tag.

7. The method according to any one of claims 1 to 6, wherein in the forming, a second capture body that specifically binds to the lipoprotein is further used for contacting the lipoprotein, the tagged sterol, and the first capture body.

8. The method according to claim 7, wherein in the forming, the lipoprotein comprising the tagged sterol is contacted with the second capture body, and then a complex of the lipoprotein comprising the tagged sterol and the second capture body is contacted with the first capture body.

9. The method according to claim 8, wherein in the forming, B/F (bound/free) separation for removing an unreacted free component is performed between
i) the contact between the lipoprotein comprising the tagged sterol and the second capture body and
ii) the contact between the complex and the first capture body.

10. The method according to any one of claims 7 to 9, wherein the second capture body is immobilized on a solid phase, and the complex of the lipoprotein comprising the tagged sterol and the second capture body is formed on the solid phase.

11. The method according to any one of claims 7 to 10, wherein the second capture body comprises an antibody that specifically binds to the lipoprotein.

12. The method according to any one of claims 1 to 11, wherein B/F (bound/free) separation for removing an unreacted free component is performed between the forming and the detecting.

## Patentansprüche

1. Verfahren zur Messung von Sterin in Lipoprotein, umfassend:
Bilden eines Komplexes durch Inkontaktbringen von Lipoprotein in einer Probe, einem mit einem Tag versehenen Sterin und einem ersten Einfangkörper, der spezifisch an das Tag bindet und eine Markierungssubstanz umfasst, miteinander,
wobei der Komplex Folgendes umfasst: das das mit einem Tag versehene Sterin umfassende Lipoprotein und den ersten Einfangkörper; und
Nachweisen eines durch die in dem Komplex enthaltene Markierung erzeugten Signals,
wobei bei dem mit einem Tag versehenen Sterin das Tag in C3-Stellung eines Steringrundgerüsts addiert ist, wobei es sich bei dem mit einem Tag versehenen Sterin um mit einem Tag versehenes Cholesterin handelt und wobei es sich bei dem Lipoprotein um Lipoprotein hoher Dichte handelt und wobei es sich bei dem Tag und dem ersten Einfangkörper um eine Kombination handelt, die aus einem Antigen und einem Antikörper, der das Antigen erkennt,
einem Hapten und einem Anti-Hapten-Antikörper, einem Peptid oder einem Protein und einem Aptamer, das sie erkennt, einem Liganden und einem Rezeptor davon, einem Oligonukleotid und einem einen komplementären Strang davon aufweisenden Oligonukleotid, Biotin oder einem Biotinanalog und einer Biotin-Bindungsstelle, einem Histidin-Tag und Nitrilotriessigsäure, die ein Chelat mit einem Nickel-Ion bildet (Ni-NTA) sowie Glutathion-S-Transferase (GST) und Glutathion ausgewählt ist.

2. Verfahren nach Anspruch 1, wobei
das mit einem Tag versehene Cholesterin durch die folgende Formel (I) wiedergegeben wird: 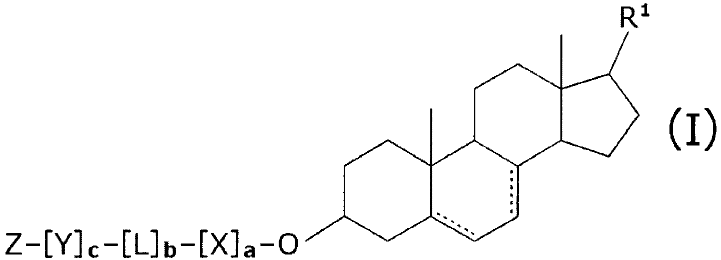
wobei Doppellinien aus einer durchgezogenen Linie und einer gestrichelten Linie jeweils unabhängig eine Einfachbindung oder eine Doppelbindung darstellen;
R¹ für eine Alkylgruppe mit 1 oder mehr und 6 oder weniger Kohlenstoffatomen, die einen Substituenten aufweisen kann, oder eine Alkenylgruppe mit 2 oder mehr und 6 oder weniger Kohlenstoffatomen, die einen Substituenten aufweisen kann, steht;
X und Y gleich oder verschieden sind und durch -R²-NH-, -NH-R²-, -R²-(C=O)-NH-, -(C=O)-NH-R²-, -R²-NH-(C=O)-, -NH-(C=O) -R²-, -R²- (C=O) -, - (C=O) -R²-, -R²- (C=O) -O-, - (C=O)-O-R²-, -R²-O-(C=O)-, -O-(C=O)-R²-, -R²-(C=S)-NH-, -(C=S)-NH-R²-, -R²-NH-(C=S)-, -NH- (C=S)-R²-, -R²-O-, -O-R²-, -R²-S- oder -S-R²- dargestellt sind und R² jeweils unabhängig für eine Atombindung, eine Alkylengruppe mit 1 oder mehr und 10 oder weniger Kohlenstoffatomen, die einen Substituenten aufweisen kann, eine Arylengruppe oder Heteroarylengruppe mit 6 oder mehr und 12 oder weniger Kohlenstoffatomen, die einen Substituenten aufweisen kann, oder eine Cycloalkylengruppe oder Heterocycloalkylengruppe mit 3 oder mehr und 8 oder weniger Kohlenstoffatomen, die einen Substituenten aufweisen kann, steht;
L durch - (CH₂)_{d}-[R³-(CH₂)ₑ]_{f}- oder -[(CH₂)ₑ-R³]_{f}-(CH₂)_{d}-dargestellt ist und R³ für ein Sauerstoffatom, ein Schwefelatom, -NH-, -NH-(C=O)-, -(C=O)-NH- oder eine Atombindung steht;
Z für ein Tag steht;
a und c gleich oder verschieden sind und für eine ganze Zahl von 0 oder größer und 6 oder kleiner stehen;
b für 0 oder 1 steht;
d und e gleich oder verschieden sind und für eine ganze Zahl von 0 oder größer und 12 oder kleiner stehen; und
f für eine ganze Zahl von 0 oder größer und 24 oder kleiner steht.

3. Verfahren nach Anspruch 2, wobei in der Formel (I) a für 0 oder 1 steht, b und c für 1 stehen, X durch -NH-(C=O)-R²- dargestellt ist, Y durch -R²-(C=O)-NH dargestellt ist, R² jeweils unabhängig für eine Alkylengruppe mit 1 oder mehr und 6 oder weniger Kohlenstoffatomen steht und keinen Substituenten aufweist, L durch -[(CH₂)₂-O]_{f}-(CH₂)_{d}- dargestellt ist, d für eine ganze Zahl von 1 oder größer und 6 oder kleiner steht und f für eine ganze Zahl von 0 oder größer und 24 oder kleiner steht.

4. Verfahren nach einem der Ansprüche 1 bis 3, wobei es sich bei dem Tag um eine Biotingruppe handelt.

5. Verfahren nach einem der Ansprüche 1 bis 4, wobei das mit einem Tag versehene Cholesterin durch die folgende Formel (II): 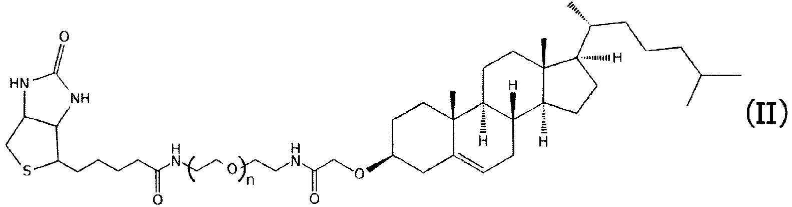
wobei n für eine ganze Zahl von 1 oder größer und 23 oder kleiner steht,
oder die folgende Formel (III): 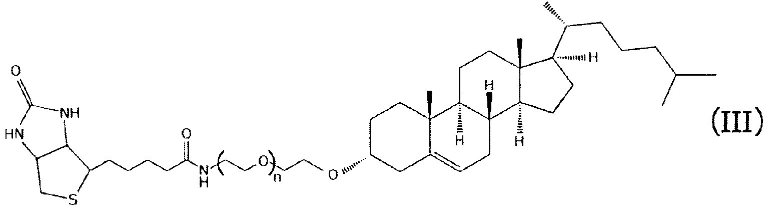
wobei n für eine ganze Zahl von 1 oder größer und 7 oder kleiner steht,
oder die folgende Formel (IV): 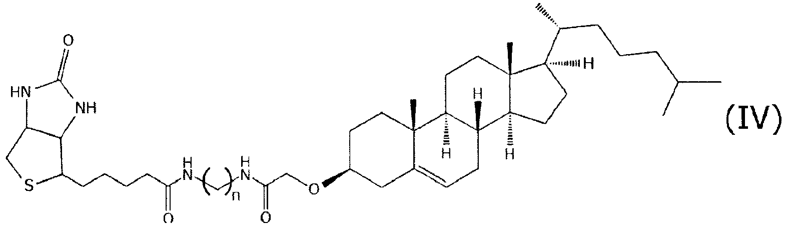
wobei n für eine ganze Zahl von 2 oder größer und 5 oder kleiner steht, wiedergegeben wird.

6. Verfahren nach einem der Ansprüche 1 bis 5, wobei es sich bei dem ersten Einfangkörper um einen markierten Antikörper, markiertes Avidin oder markiertes Streptavidin handelt, wobei der erste Einfangkörper spezifisch an das Tag bindet.

7. Verfahren nach einem der Ansprüche 1 bis 6, wobei beim Bilden ein zweiter Einfangkörper, der spezifisch an das Lipoprotein bindet, weiter zum Inkontaktbringen des Lipoproteins, des mit einem Tag versehenen Sterins und des ersten Einfangkörpers verwendet wird.

8. Verfahren nach Anspruch 7, wobei beim Bilden das das mit einem Tag versehene Sterin umfassende Lipoprotein mit dem zweiten Einfangkörper in Kontakt gebracht wird und dann ein Komplex des das mit einem Tag versehene Sterin umfassenden Lipoproteins und des zweiten Einfangkörpers mit dem ersten Einfangkörper in Kontakt gebracht wird.

9. Verfahren nach Anspruch 8, wobei beim Bilden eine B/F(gebunden/frei)-Trennung zur Entfernung einer nicht umgesetzten freien Komponente zwischen
i) dem Kontakt zwischen dem das mit einem Tag versehene Sterin umfassenden Lipoprotein und dem zweiten Einfangkörper und
ii) dem Kontakt zwischen dem Komplex und dem ersten Einfangkörper durchgeführt wird.

10. Verfahren nach einem der Ansprüche 7 bis 9, wobei der zweite Einfangkörper an einer Festphase immobilisiert ist und der Komplex des das mit einem Tag versehene Sterin umfassenden Lipoproteins und des zweiten Einfangkörpers an der Festphase gebildet wird.

11. Verfahren nach einem der Ansprüche 7 bis 10, wobei der zweite Einfangkörper einen Antikörper umfasst, der spezifisch an das Lipoprotein bindet.

12. Verfahren nach einem der Ansprüche 1 bis 11, wobei eine B/F(gebunden/frei)-Trennung zur Entfernung einer nicht umgesetzten freien Komponente zwischen dem Bilden und dem Nachweisen durchgeführt wird.

## Revendications

1. Procédé pour la mesure de stérol dans une lipoprotéine, comprenant :
la formation d'un complexe par la mise en contact les uns avec les autres : d'une lipoprotéine présente dans un échantillon ; d'un stérol étiqueté ; et d'un premier corps de capture qui se lie spécifiquement à l'étiquette et qui comprend une substance de marquage,
le complexe comprenant : la lipoprotéine comprenant le stérol étiqueté ; et le premier corps de capture ; et
la détection d'un signal généré par la substance de marquage comprise dans le complexe,
dans lequel, dans le stérol étiqueté, l'étiquette est ajoutée en position C3 d'un squelette de stérol, dans lequel le stérol étiqueté est le cholestérol étiqueté et dans lequel la lipoprotéine est une lipoprotéine de haute densité et dans lequel l'étiquette et le premier corps de capture sont une combinaison choisie parmi un antigène et un anticorps qui reconnaît l'antigène, un haptène et un anticorps anti-haptène, un peptide ou une protéine et un aptamère qui les reconnaît, un ligand et un récepteur de celui-ci, un oligonucléotide et un oligonucléotide ayant un brin complémentaire de celui-ci, la biotine ou un analogue de la biotine et un site de liaison à la biotine, une étiquette histidine et l'acide nitrilotriacétique qui forme un chélate avec un ion nickel (Ni-NTA) et la glutathion-S-transférase (GST) et le glutathion.

2. Procédé selon la revendication 1, dans lequel
le cholestérol étiqueté est représenté par la formule (I) suivante : 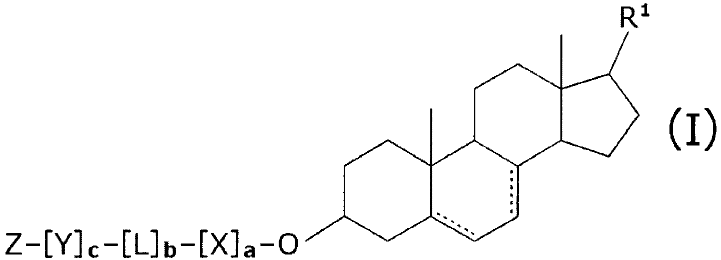
où les traits doubles constitués d'un trait continu et d'un trait discontinu représentent chacun indépendamment une liaison simple ou une double liaison ;
R¹ est un groupe alkyle ayant un nombre d'atomes de carbone supérieur ou égal à 1 et inférieur ou égal à 6 qui peut porter un substituant ou un groupe alcényle ayant un nombre d'atomes de carbone supérieur ou égal à 2 et inférieur ou égal à 6 qui peut porter un substituant ;
X et Y sont identiques ou différents et sont représentés par -R²-NH-, -NH-R²-, -R²- (C=O)-NH-, -(C=O)-NH-R²-, -R²-NH-(C=O)-, -NH-(C=O)-R²-, -R²-(C=O)-, -(C=O)-R²-, -R²-(C=O)-O-, -(C=O)-O-R²-, -R²-O-(C=O)-, -O-(C=O)-R²-, -R²-(C=S) -NH-, - (C=S)-NH-R²-, -R²-NH-(C=S) -, -NH- (C=S)-R²-, - R²-O-, -O-R²-, -R²-S- ou -S-R²- et chaque R² est indépendamment une liaison atomique, un groupe alkylène ayant un nombre d'atomes de carbone supérieur ou égal à 1 et inférieur ou égal à 10 qui peut porter un substituant, un groupe arylène ou un groupe hétéroarylène ayant un nombre d'atomes de carbone supérieur ou égal à 6 et inférieur ou égal à 12 qui peut porter un substituant ou un groupe cycloalkylène ou hétérocycloalkylène ayant un nombre d'atomes de carbone supérieur ou égal à 3 et inférieur ou égal à 8 qui peut porter un substituant ;
L est représenté par -(CH₂)_{d}-[R³-(CH₂)ₑ]_{f}- ou -[(CH₂)ₑ-R³]_{f}-(CH₂)_{d}- et R³ est un atome d'oxygène, un atome de soufre, -NH-, -NH-(C=O)-, -(C=O)-NH- ou une liaison atomique ;
Z est une étiquette ;
a et c sont identiques ou différents et sont chacun un nombre entier supérieur ou égal à 0 et inférieur ou égal à 6 ;
b vaut 0 ou 1 ;
d et e sont identiques ou différents et sont chacun un nombre entier supérieur ou égal à 0 et inférieur ou égal à 12 ; et
f est un nombre entier supérieur ou égal à 0 et inférieur ou égal à 24.

3. Procédé selon la revendication 2, dans lequel, dans la formule (I), a vaut 0 ou 1, b et c valent 1, X est représenté par -NH-(C=O)-R²-, Y est représenté par -R²-(C=O)-NH-, les R² sont chacun indépendamment un groupe alkylène ayant un nombre d'atomes de carbone supérieur ou égal à 1 et inférieur ou égal à 6 et ne portant pas de substituant, L est représenté par -[(CH₂)₂-O]ᵣ-(CH₂)_{d}- , d est un nombre entier supérieur ou égal à 1 et inférieur ou égal à 6 et f est un nombre entier supérieur ou égal à 0 et inférieur ou égal à 24.

4. Procédé selon l'une quelconque des revendications 1 à 3, dans lequel l'étiquette est un groupe de la biotine.

5. Procédé selon l'une quelconque des revendications 1 à 4, dans lequel le cholestérol étiqueté est représenté par la formule (II) suivante : 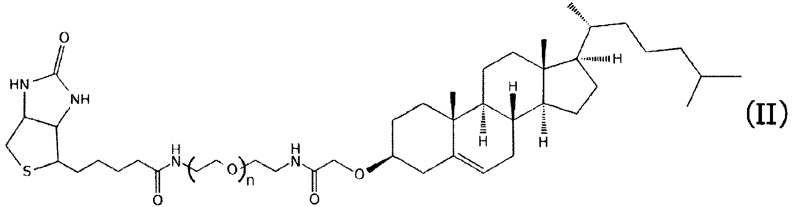
où n est un nombre entier supérieur ou égal à 1 et inférieur ou égal à 23,
ou la formule (III) suivante : 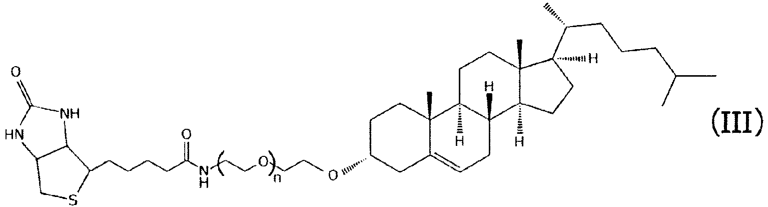
où n est un nombre entier supérieur ou égal à 1 et inférieur ou égal à 7,
ou la formule (IV) suivante : 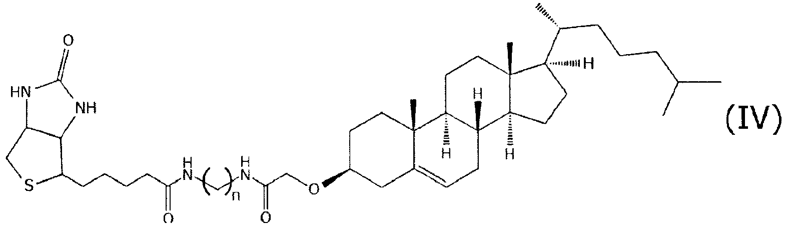
où n est un nombre entier supérieur ou égal à 2 et inférieur ou égal à 5.

6. Procédé selon l'une quelconque des revendications 1 à 5, dans lequel le premier corps de capture est un anticorps marqué, l'avidine marquée ou la streptavidine marquée, le premier corps de capture se liant spécifiquement à l'étiquette.

7. Procédé selon l'une quelconque des revendications 1 à 6, dans lequel, lors de la formation, un second corps de capture qui se lie spécifiquement à la lipoprotéine est en outre utilisé pour la mise en contact de la lipoprotéine, du stérol étiqueté et du premier corps de capture.

8. Procédé selon la revendication 7, dans lequel, lors de la formation, la lipoprotéine comprenant le stérol étiqueté est mise en contact avec le second corps de capture, puis un complexe de la lipoprotéine comprenant le stérol étiqueté et du second corps de capture est mis en contact avec le premier corps de capture.

9. Procédé selon la revendication 8, dans lequel, lors de la formation, une séparation B/F (lié/libre) pour l'élimination d'un composant libre n'ayant pas réagi est effectuée entre
i) la mise en contact entre la lipoprotéine comprenant le stérol étiqueté et le second corps de capture et
ii) la mise en contact entre le complexe et le premier corps de capture.

10. Procédé selon l'une quelconque des revendications 7 à 9, dans lequel le second corps de capture est immobilisé sur une phase solide et le complexe de la lipoprotéine comprenant le stérol étiqueté et du second corps de capture est formé sur la phase solide.

11. Procédé selon l'une quelconque des revendications 7 à 10, dans lequel le second corps de capture comprend un anticorps qui se lie spécifiquement à la lipoprotéine.

12. Procédé selon l'une quelconque des revendications 1 à 11, dans lequel une séparation B/F (lié/libre) pour l'élimination d'un composant libre n'ayant pas réagi est effectuée entre la formation et la détection.
